# EUROPEAN PATENT SPECIFICATION

(11) **EP 0 540 099 B1**
(45) Date of publication and mention of the grant of the patent: **17.04.1996**
(21) Application number: 92203263.6
(22) Date of filing: 23.10.1992
(51) Int. Cl.: C07D 491/22, A61K 31/33, C07D 317/66, C07D 319/18

(54) **Water soluble camptothecin derivatives**
Wasserlösliche Camptothecinderivate
Dérivés de camptothécine solubles dans l'eau

(30) Priority: 29.10.1991 US 784275; 28.01.1992 US 826729
(43) Date of publication of application: 05.05.1993
(73) Proprietor: GLAXO WELLCOME INC., Research Triangle Park, North Carolina 27709 (US)
(72) Inventor: Luzzio, Michael Joseph, Durham, North Carolina 27707 (US); Besterman, Jeffrey M., Durham, North Carolina 27713 (US); Evans, Michael G., Pittsboro, North Carolina 27312 (US); Myers, Peter Leslie, Chapel Hill, North Carolina 27514 (US)
(74) Representative: Filler, Wendy Anne

(56) References cited:
- EP-A- 0 321 122
- JOURNAL OF MEDICINAL CHEMISTRY. vol. 34, no. 1, 1991, WASHINGTON US pages 98 - 107 W.D. KINGSBURY ET AL. 'Synthesis of water-soluble (aminoalkyl)camptothecin analogues: inhibition of topoisomerase I and antitumor activity'

## Description

The present invention relates to water soluble, camptothecin derivatives substituted in the 7 position, their use in the treatment of tumors and methods of their preparation.

### BACKGROUND OF THE INVENTION

Camptothecin, a natural, cytotoxic alkaloid, is a topoisomerase I inhibitor and potent antitumor agent. It was first isolated from the leaves and bark of the Chinese plant, *Camptotheca accuminata,* by Wall, *et al.* (*J. Am. Chem. Soc.,* **88** 3888 (1966)).

As depicted, camptothecin is a fused ring system, composed of a quinoline (A and B), fused to a pyrrolidine ring (C), fused to an alpha-pyridone ring (D) which in turn is fused to a lactone ring (E). 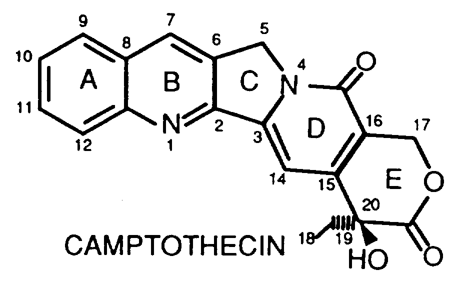 It has an asymmetric carbon at the 20 position making two enantiomeric forms possible. However, the natural occurring compound is found in the "S" configuration as shown above.

Cytotoxic agents are often employed to control or eradicate tumors *i.e*., they are chemotherapeutic agents. Camptothecin's cytotoxic activity is thought to be directly related to camptothecin's potency as a topoisomerase inhibitor. [For detailed explanations of the topoisomerase function see A. Lehninger, *Principles of Biochemistry,* 813, Worth Publishers, New York (1982); L. F. Liu, "DNA Topoisomerases," *CRC Critical Review in Biochemistry,* 1-24, **15** (1983) and H Vosberg, "DNA Topoisomerases: Enzymes that Control DNA Conformation," *Current Topics in Microbiology and Immunology,* 19, Springer-Verlag, Berlin (1985).] In particular, camptothecin has been shown to be effective in the treatment of leukemia (L-1210) and certain solid tumors in laboratory animals, *e.g., see Chem. Rev.* **23**, 385 (1973) and *Cancer Treat. Rep.,* **60**, 1007 (1967).

Unfortunately, in the clinic camptothecin's promise as an effective antitumor agent has not been completely fulfilled. Camptothecin is essentially insoluble in physiologically compatible, aqueous media, and must be modified to make it sufficiently soluble for parenteral administration, a preferred mode for antitumor treatment. It can be made soluble by forming its sodium salt, that is, by opening the lactone with sodium hydroxide (see F.M. Muggia, *et al*., *Cancer Chemotherapy Reports,* pt. 1, **56**, No.4, 515 (1972)). However, M. C. Wani, *et al., J. Med. Chem,* **23**, 554 (1980), reported that the alpha-hydroxy lactone moiety of ring E is an absolute requirement for antitumor activity.

In the art there are examples of modifications and derivatives of camptothecin prepared to improve its solubility in water. Although many of these derivatives were active in *in vitro* and in early animal studies using leukemia (L-1210) models, they were disappointing in chronic, animal models involving implanted solid tumors.

Miyasaka, *et al.*, U.S. Patent No. 4,399,282, discloses a group of camptothecin derivatives substituted at the 7 position with, *inter alia,* hydroxymethyl and alkoxymethyl. Further, Miyasaka, *et. al.* in U.S. patent No. 4,399,276 discloses camptothecin-7-aldehyde and certain related aldehyde derivatives such as acetals, oximes and hyrazones. More recently, Vishnuvajjala, *et al.*, in U.S. Patent No. 4,943,579, claimed a series of water-soluble camptothecin derivatives with substituents on the A ring as does Boehm, *et al.,* European Patent Application 0 321 122 A2. Other examples of derivatives of camptothecin include Miyasaka, *et al.*, U.S. Patent No. 4,473,692 and No. 4,545,880; and W. Kingsbury, *et al.*, *J Med. Chem.*, **34**, 98 (1991). None of these references reported compounds with antitumor activity greater than that of camptothecin itself.

Wani and co-workers reported that 10, 11-methylenedioxycamptothecin is more potent than unsubstituted camptothecin (see M. C. Wani, *et al., J. Med. Chem,* **29**, 2358 (1986) and **30**, 2317 (1987)). However, its water solubility is as poor as camptothecin which seriously limits its clinical utility.

We have now found water-soluble analogs of camptothecin with good topoisomerase I inhibitory activity *in vitro* and impressive antitumor activity *in vivo.*

### SUMMARY OF THE INVENTION

One aspect of the present invention are the water-soluble camptothecin analogs of formula (I), 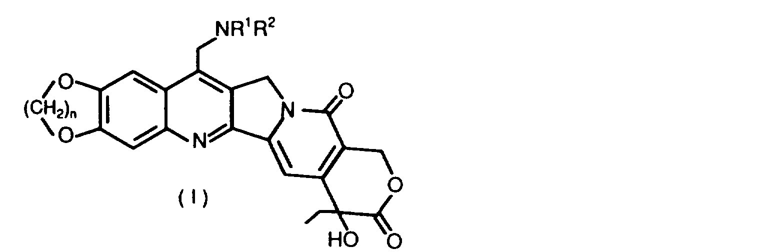 wherein:
n represents the integer 1 or 2; and
i) R¹ and R represent independently, hydrogen, lower alkyl, (C₃₋₇)cycloalkyl, (C₃₋₇)cycloalkyl lower alkyl, lower alkenyl, hydroxy lower alkyl, lower alkoxy lower alkyl;
ii) R¹ represents hydrogen, lower alkyl, (C₃₋₇)cycloalkyl, (C₃₋₇)cycloalkyl lower alkyl, lower alkenyl, hydroxy lower alkyl or lower alkoxy lower alkyl, and
   R represents-COR³,
   wherein:
   R³ represents hydrogen, lower alkyl, perhalo-lower alkyl, (C₃₋₇)cydoalkyl, (C₃₋₇)cydoalkyl lower alkyl, lower alkenyl, hydroxy lower alkyl, lower alkoxy, lower alkoxy lower alkyl; or
iii) R¹ and R taken together with the linking nitrogen form a saturated 3 to 7 atom heterocyclic group of formula (IA) 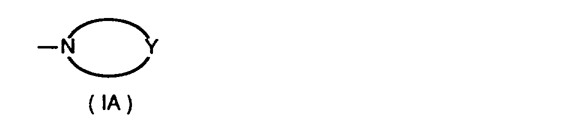
   wherein:
   Y represents O, S, CH₂ or NR⁴
   wherein:
   R⁴ represents hydrogen, lower alkyl, perhalo-lower alkyl, aryl, aryl substituted with one or more
   lower alkyl, halogen, nitro, amino, lower alkyl amino, perhalo-lower alkyl, hydroxy lower alkyl, lower alkoxy, lower alkoxy lower alkyl groups; or
   -COR⁵,
   wherein:
   R⁵ represents hydrogen, lower alkyl, perhalo-lower alkyl, lower alkoxy, aryl, aryl substituted with one or more
   lower alkyl, perhalo-lower alkyl, hydroxy lower alkyl, lower alkoxy lower alkyl groups; and
   the pharmaceutically acceptable salts thereof.

Pharmaceutically acceptable salts include, but are not limited to salts with inorganic acids such hydrochloride, sulfate, phosphate, diphosphate, hydrobromide and nitrate or salts with an organic acid such as acetate, malate, maleate, fumarate, tartrate, succinate, citrate, lactate, methanesulfonate, p-toluenesulfonate, palmoate, salicylate and stearate. Other acids such as oxalic, while not in themselves pharmaceutically acceptable, may be useful as intermediates in obtaining the compounds of the invention and their pharmaceutically aceptable salts.

The lactone ring, ring E, may be opened by alkali metal or alkaline-earth metal bases, for example sodium hydroxide or calcium hydroxide, to form alkali metal or alkaline-earth metal salts of the corresponding open E ring form of the compounds of formula (I). Because of its better solubility in water, the open E ring form may advantageously be purified by conventional recrystallization techniques. Accordingly, said open E ring form may then be used as an intermediate to form the compounds of formula (I), for example by treatment with acid, *e.g.,* hydrochloric acid, and thereby produce a purified form of the compounds of formula (I).

As noted above, the camptothecin moiety has an asymmetric carbon atom at the 20 position making two enantiomeric forms, *i.e.,* "R" and "S" configurations, possible. This invention includes both enantiomeric forms and any combinations of these forms. For simplicity, where no specific configuration at the 20 position is depicted in the structural formulas, it is to be understood that both enantiomeric forms and mixtures thereof are represented. Unless noted otherwise, the nomenclature convention, "(R,S)", denotes a racemic (approximately equal portion) mixture of the R and S enantiomers while "(R)" and "(S)" denote essentially optically pure R and S enantiomers respectively. Also included in the invention are other forms of the compound of formula (I), such as solvates, hydrates, polymorphs and the like.

Another aspect of the invention is a method of inhibiting topoisomerase Type I in mammalian cells comprising administering to a patient a topoisomerase inhibiting amount of a compound of formula (I), and a method of treating a tumor in a mammal comprising administering to a mammal bearing a tumor, an effective antitumor amount of a compound of formula (I). A further aspect comprises pharmaceutical formulations containing a compound of formula (I) as an active ingredient. Methods of preparation of the compounds of formula (I) and the associated novel chemical intermediates used in the synthesis, as taught herein, are also within the scope of the present invention.

### DETAILED DESCRIPTION OF THE INVENTION

### Compounds

As used herein the term "lower" in reference to alkyl and alkoxy means 1-6 carbons and in reference to alkenyl means 3-6 carbons (provided that the double bond is not attached to the carbon which is attached to the nitrogen). The term "perhalo" means all hydrogens have been replaced with a halogen, for example, perhalo lower-alkyl, *e.g*., trifluoromethyl. The term "aryl" means phenyl or napthyl.

Particular compounds of formula (I) are those wherein:
R¹ and R represent:
i) independently; hydrogen, (C₁₋₄)alkyl, (C₃₋₇)cycloalkyl, (C₃₋₇)cycloalkyl (C₁₋₄)alkyl, (C₃₋₄)alkenyl, hydroxy (C₁₋₄)alkyl, (C₁₋₄)alkoxy (C₁₋₄)alkyl, or
ii) taken together with the nitrogen form aziridine, azetidine, pyrrolidine, piperidine, hexamethylenimine, imidazolidine, pyrazolidine, isoxazolidine, piperazine, N-methylpiperazine, homopiperazine, N-methylhomopiperazine, thiazolidine, isothiazolidine,morpholine or thiomorpholine.
   One preferred group of compounds according to the invention are the compounds of formula (I) wherein:
   n represents the integer 1 or 2; and
   i) R¹ and R represent independently, hydrogen, lower alkyl (e.g. methyl, ethyl) or hydroxy lower alkyl (e.g. hydroxyethyl);
   ii) R¹ represents hydrogen and R represents -COR³, wherein R³ represents perhalo-lower alkyl(e.g. trifluoromethyl); or
   iii) R¹ and R taken together with the linking nitrogen form azetidine, pyrrolidine, piperidine, morpholine, thiomorpholine or piperazine optionally N-substituted with lower alkyl (e.g. methyl); phenyl; phenyl substituted with one or more perhalo-lower alkyl (e.g. trifluoromethyl) or lower alkoxy (e.g. methoxy) groups; or -COR⁵, wherein R⁵ represents lower alkoxy (e.g. butoxy); and
   the pharmaceutically acceptable salts thereof.
Specific compounds of formula (I) are:

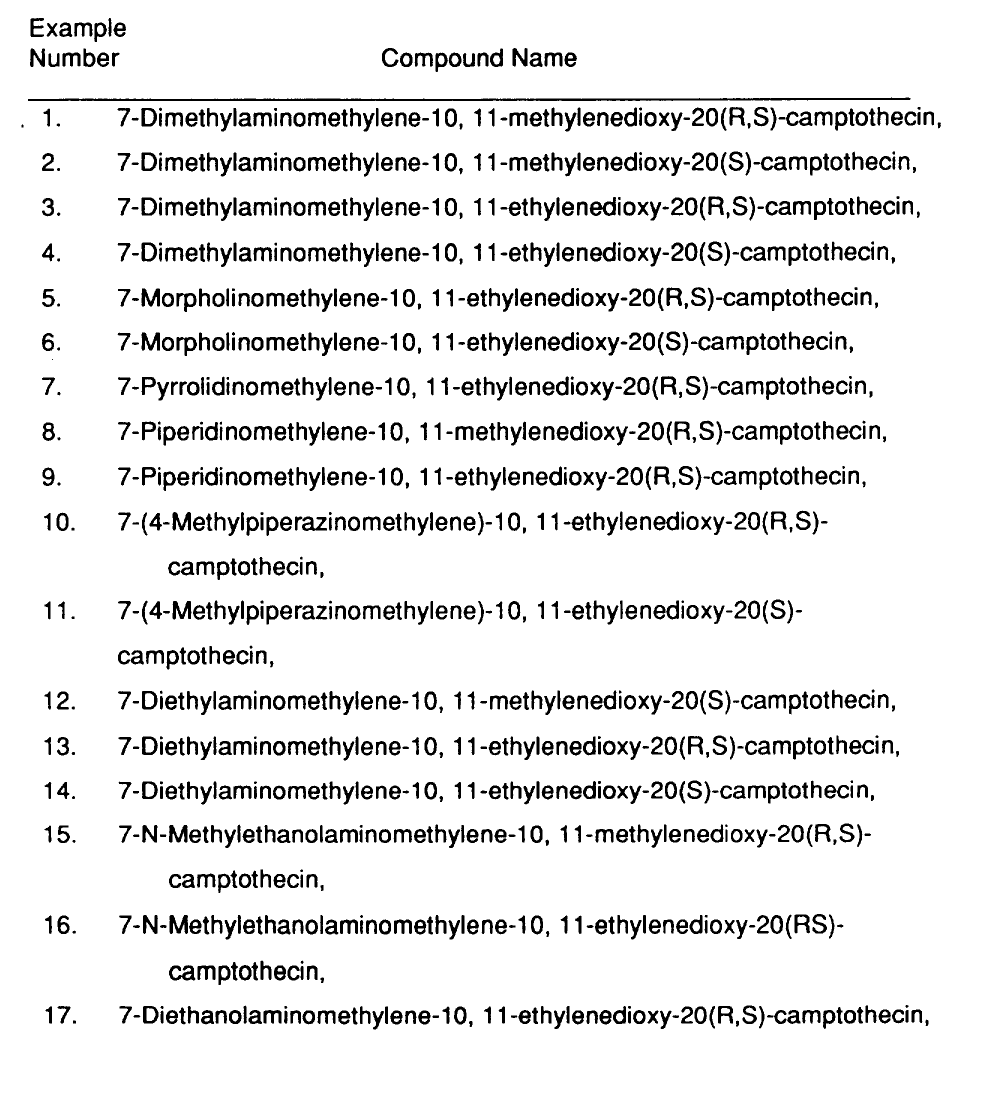

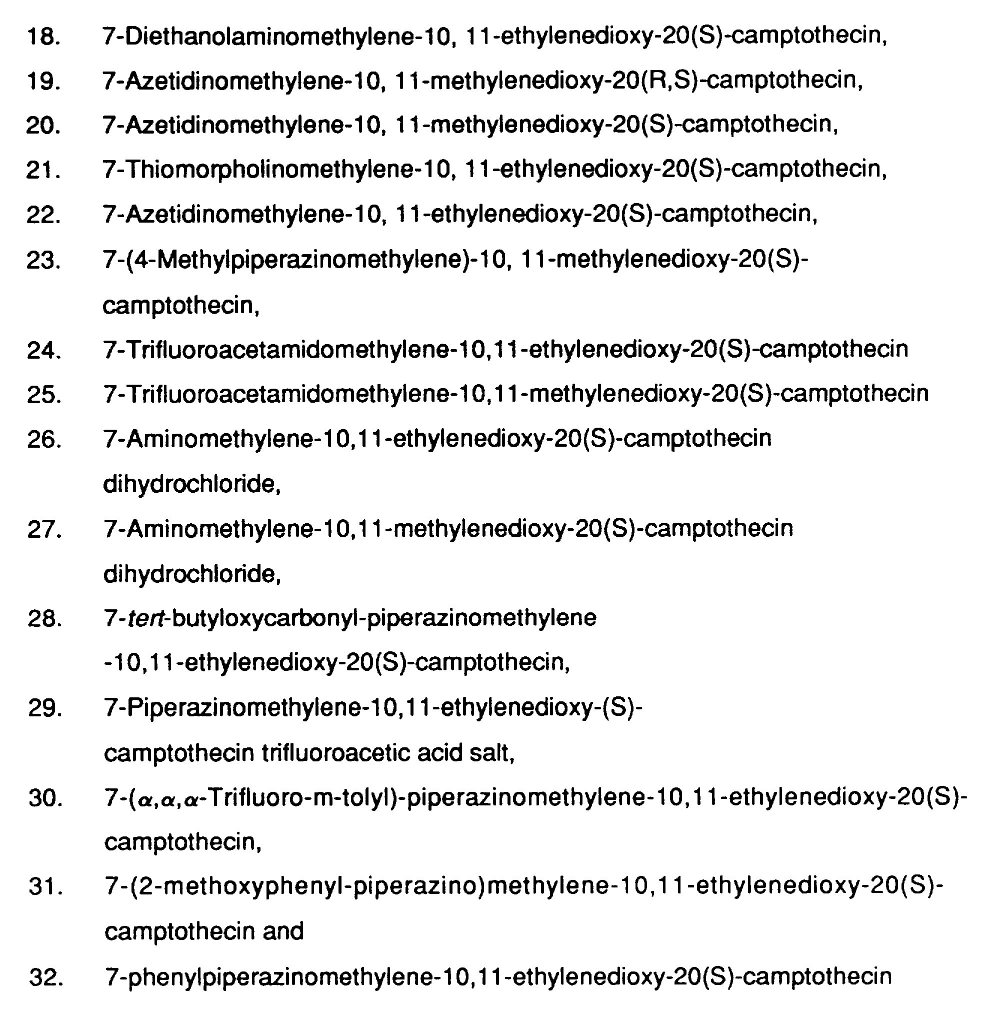

Other particular compounds of formula (I) are those of formula (I') 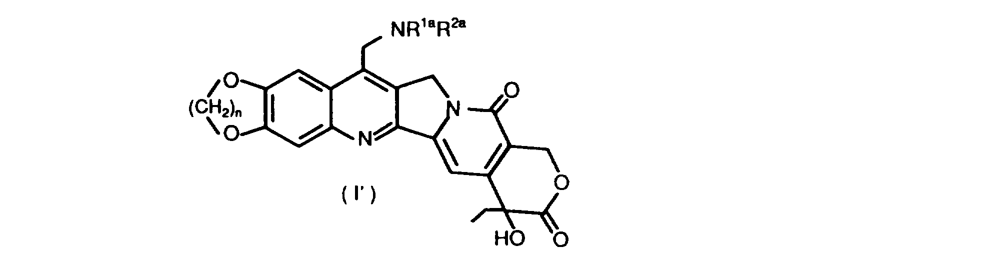 wherein:
n represents the integer 1 or 2; and
R^{1a} and R^{2a} represent:
i) independently, hydrogen, lower alkyl, (C₃₋₇) cycloalkyl, (C₃₋₇)cycloalkyl lower alkyl, lower alkenyl, hydroxy lower alkyl, lower alkoxy lower alkyl, or
ii) taken together with the linking nitrogen form a 3 to 7 atom heterocyclic group of formula (IA')
   wherein; 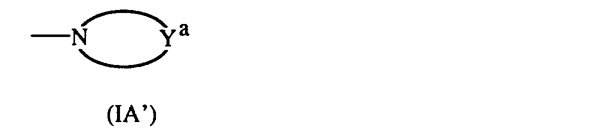 Y^{a} represents O, S, CH₂, NH or N(lower alkyl), and the pharmaceutically acceptable salts thereof.

### Preparation of Compounds

According to one general process (A) the compounds of formula I may be prepared by the procedure shown in Step 2 of Scheme I below:

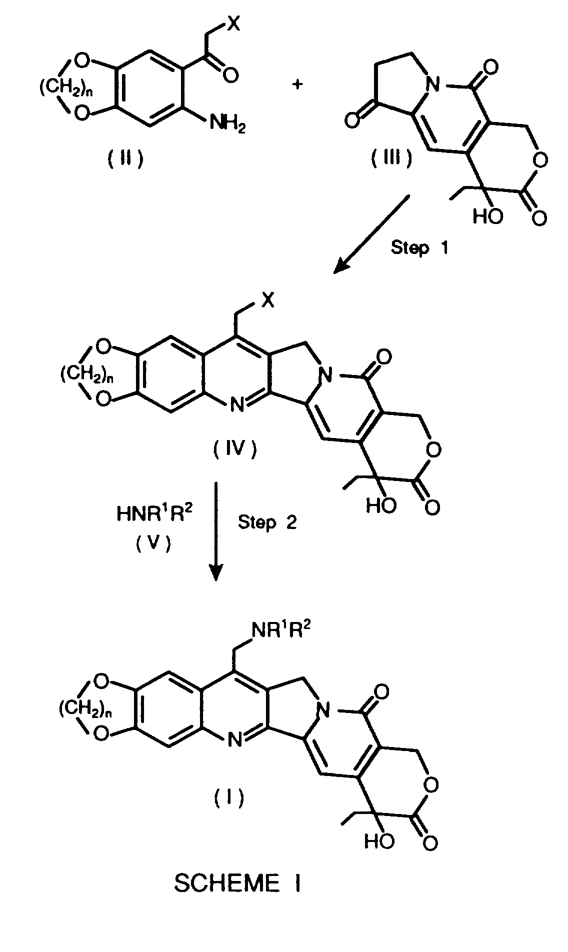

In Step 1 of Scheme I, a compound of formula (II), wherein X is a leaving group (as defined in J. March, *Advanced Organic Chemistry,* 3rd. Ed., page 179, John Wiley & Sons, New York (1985)), for example, a halogen, *e.g.*, chloro, may be reacted with a compound of formula (III) according to the method taught in US Patent 4, 894,456 (hereinafter, '456), issued January 16, 1990 to Wall *et al.,* incorporated herein by reference, to yield a compound of formula (IV).

In Step 2 (General Process A), the compounds of formula (IV) may be converted to the compounds of formula (I) by displacement of the leaving group, X, with a compound of formula (V), wherein R¹ and R are as defined for formula (I). This displacement reaction may conveniently be carried out in a solvent system, for example water, a (C₁₋₄) alkanol, a (C₂₋₄) alkylene diol, 1-hydroxy-2-methoxyethane, dimethylacetamide (DMAC), N-methylpyrrolidinone, dimethyl formamide (DMF), tetrahydrofuran (THF), dimethyl sulfoxide (DMSO), toluene or a combination of these solvents in the presence of excess amine, *i.e.,* excess compound of formula (V), with or without a base, *e.g.,* potassium carbonate.

This method is particularly useful for preparing compounds of formula (I) wherein neither R¹ nor R are hydrogen.

According to another general process (B) compounds of formula (I) may be prepared by the procedure shown in Step 2a of Scheme 1A below:

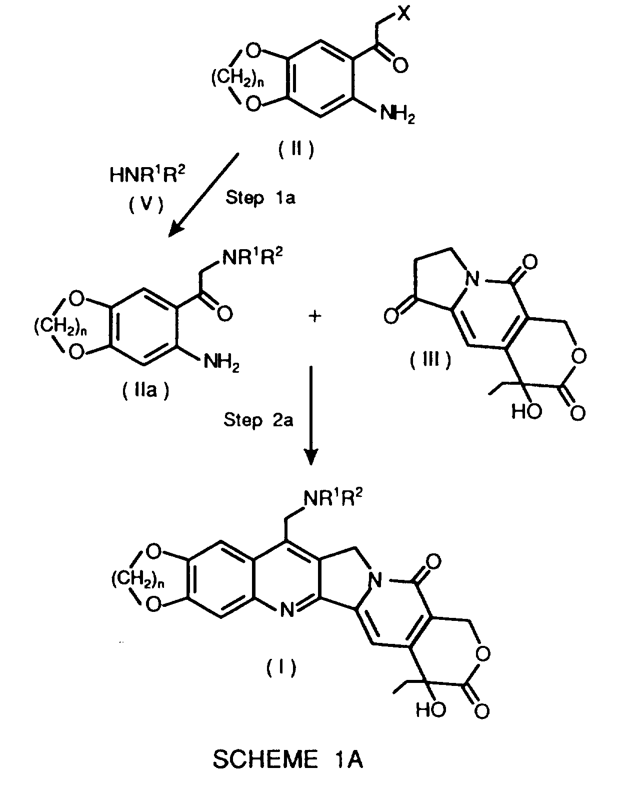

In Step 1a, a compound of formula (V) is reacted with a compound of formula (II) to yield a compound of formula (IIA), wherein R¹ and R are as defined for the compounds of formula (I). This reaction may be carried out under conditions similar to those described in general process (A) (Scheme 1, Step 2).

In Step 2a (general process B), compound of formula (IIA) is reacted with a compound of formula (III) in a similar manner to that taught above in Scheme 1, Step 1, to yield a compound of formula (I).

In a particular embodiment of process (B) the compounds of formula (I) may be prepared by the procedure shown in Scheme 1B below:

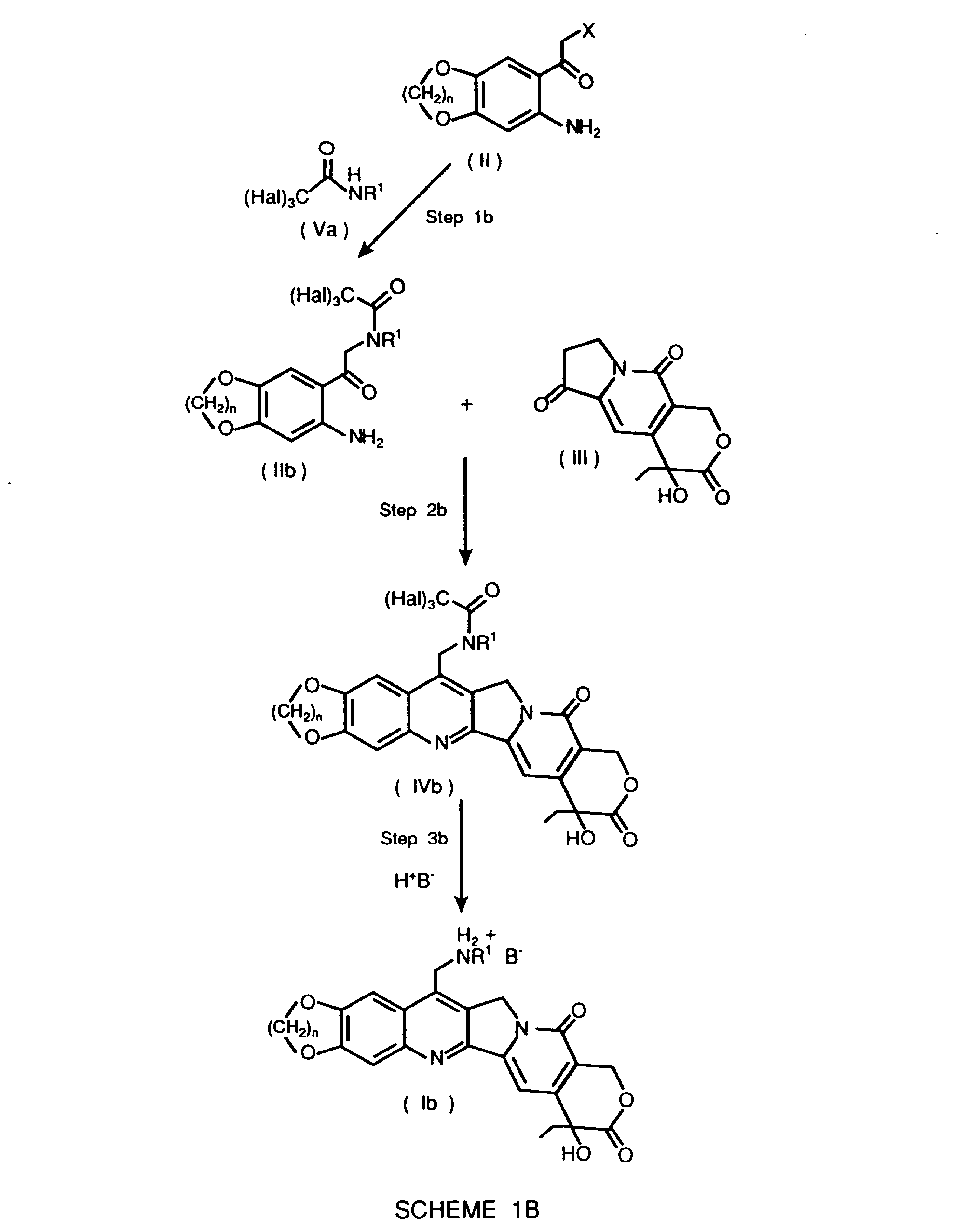

In Step 1b, a compound of formula (Va) (wherein "Hal" is halogen, *i.e*., fluoro, chloro, bromo or iodo) *e.g.*, trifluoroacetamide, is reacted with a compound of formula (II) in a polar, aprotic solvent, *e.g.,* acetonitrile, in the presence of a base soluble in the polar, aprotic solvent, *e.g.*, cesium carbonate if the solvent is acetonitrile, to yield a compound of formula (IIb).

In Step 2b, a compound of formula (IIb) is reacted with a compound of formula (III) in a similar manner to that taught in Scheme 1, Step 1, to yield a compound of formula (IVb).

In Step 3b, a compound of formula (IVb) is treated with an acid, H⁺B⁻, such as a mineral acid, *e.g.*, hydrochloric acid, to yield a compound of formula (Ib), *i.e.* salt of a compound of formula (I). The compound of formula (Ib) may be treated with a base by standard method of the art to yield the corresponding free base. The free base can then be converted by conventional means to a pharmaceutically acceptable salt if required.

This alternate method is particularly useful for preparing compounds of formula (I) wherein both R¹ and R are hydrogen or R is hydrogen.

The compounds of formula (II) may be prepared according to the procedure shown in Scheme II:

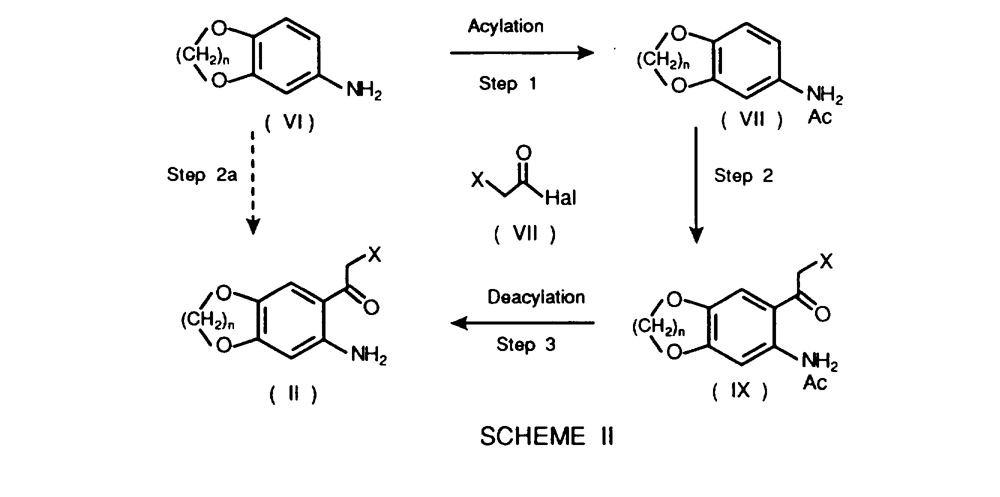

In Step 1 of Scheme II, a compound of formula (VI) is reacted with an acylating agent, for example, a (C₂₋₅)alkanoic acid halide or (C₂₋₅) alkanoic acid anhydride, *e.g.*, acetyl chloride or acetic anhydride, in the presence of a weak base, for example, potassium carbonate, in a polar, aprotic, solvent, for example, chloroform, to yield a compound of formula (VII), wherein Ac is a (C₂₋₅) acyl group.

In Step 2, a compound of formula (VII) is reacted with a compound of formula (VIII), wherein X is a leaving group as defined for the compounds of formula (IV) and Hal is halogen, in the presence of a metalic halide, *e.g*., zinc chloride, in a polar, aprotic solvent, *e.g*., nitromethane, to yield a compound of formula (IX). A compound of formula (VIII), for example, may be a haloacetyl halide, *e.g*., chloroacetyl chloride, or a haloacetonitrile, *e.g.*, chloroacetonitrile.

In Step 3, a compound of formula (II) is formed by removal of the acyl group, Ac, from a compound of formula (IX), *i.e.*, deacylation, by methods known in the art, such as those taught in T. Green, *Protective Groups in Organic Chemistry,* Chap. 7, John Wiley, New York (1981). For example, a compound of formula (IX) may be heated at reflux in concentrated hydrochloric acid, and the resulting salt neutralized with a base, *e.g.*, sodium hydroxide, to yield a compound of formula (II).

In Step 2a of Scheme II, when the compound of formula (VI) is the ethylenedioxy compound, *i.e.*, n is equal to 2, it may be reacted directly with a compound of formula (VIII), without first protecting the amino group by acylation, to yield the corresponding compound of formula (II).

Alternatively compounds of formula (II) may be prepared according to the method taught by T. Sugasawa, *et al., J. Org. Chem.,* **44**, 578 (1979).

The compound of formula (III) may be prepared according to the procedure of Wall, *et al*., '456, at column 11, starting at line 30. It is apparent from Scheme 1 that the configuration of the asymmetric carbon of the compound of formula (III) will govern the configuration of the compounds of formula (I). The racemic compound of formula (III) can be resolved into either of its enantiomers by the method of Wani, *et al.*, in United States Patent 5,053,512, (hereinafter, "'512") incorporated herein by reference.

The novel, intermediate compounds of formulas (II), (IIA) and (IV) are within the scope of this invention.

According to another general process (D), a compound of formula (I) according to the invention may be converted into another compound of the invention using conventional procedures.

Thus, for example, a compound of formula (I) wherein one or more of R¹ and R represent a hydrogen atom, may be alkylated using conventional techniques. The reaction may be effected using a suitable alkylating agent such as an alkyl halide, an alkyl tosylate or a dialkylsulphate. The alkylation reaction may conveniently be carried out in an organic solvent such as an amide, e.g. dimethylformamide, or an ether, e.g. tetrahydrofuran, preferably in the presence of a base. Suitable bases include, for example, alkali metal hydrides, such as sodium hydride, alkali metal carbonates, such as sodium carbonate, or alkali metal alkoxides such as sodium or potassium methoxide, ethoxide or t-butoxide. The alkylation reaction is conveniently carried out at a temperature of from 25 to 100°C.

Alternatively a compound of formula (I) wherein one or more of R¹ and R represents a hydrogen atom may be converted to another compound of formula (I) by reductive alkylation. Reductive alkylation with an appropriate aldehyde or ketone may be effected using an alkaline earth metal borohydride or cyanoborohydride. The reaction may be effected in an aqueous or non-aqueous reaction medium, conveniently in an alcohol, e.g. methanol or ethanol or an ether, e.g. dioxan or tetrahydrofuran, optionally in the presence of water. The reaction may conveniently be carried out at a temperature in the range of 0 to 100°C, preferably 5 to 50°C.

Alternatively a compound of formula (I) wherein one or more of R¹ and R represents a lower alkenyl group may be converted to another compound of formula (I) wherein R¹ or R represents a lower alkyl group. Reduction may conveniently be effected in the presence of hydrogen and a metal catalyst, for example Raney nickel or a nobel metal catalyst such as palladium, platinum, platinum oxide or rhodium, which may be supported for example on charcoal. The reaction may be effected in a solvent such as an alcohol, for example ethanol and conveniently at a temperature of from -10 to +50°C, preferably 20 to 30°C.

Alternatively compounds of formula (I) wherein R represents a hydroxyalkyl group may be prepared by reduction of a compound of formula (I) wherein R represents a group COR³ wherein R³ represents a lower alkoxy group. The reduction may conveniently be carried out using a suitable hydride reducing agent, e.g. lithium aluminium hydride or lithium triethylborohydride, in a suitable solvent such as an ether, e.g. diethyl ether, tetrahydrofuran or dioxan, or a hydrocarbon such as toluene. The reaction is conveniently conducted at a temperature of -78 to 100°C, preferably at about 0°C.

According to another general process (E), a compound of formula (I) according to the invention, or a salt thereof may be prepared by subjecting a protected derivative of formula (I) or a salt thereof to reaction to remove the protecting group or groups.

Thus, at an earlier stage in the preparation of a compound of formula (I) or a salt thereof it may have been necessary and/or desirable to protect one or more sensitive groups in the molecule to prevent undesirable side reactions.

The protecting groups used in the preparation of compounds of formula (I) may be used in conventional manner. See for example 'Protective Groups in Organic Chemistry' Ed.J.F.W.McOmie (Plenum Press 1973) or 'Protective Groups in Organic Synthesis' by Theodora W Greene (John Wiley and Sons 1981).

Conventional amino protecting groups may include for example aralkyl groups, such as benzyl, diphenylmethyl or triphenylmethyl groups; and acyl groups such as N-benzyloxycarbonyl or t-butoxycarbonyl. Thus, compounds of general formula (I) wherein one or more of the groups R¹ and R represent hydrogen may be prepared by deprotection of a corresponding protected compound.

Hydroxy groups may be protected, for example, by aralkyl groups, such as benzyl, diphenylmethyl or triphenylmethyl groups, acyl groups, such as acetyl, silicon protecting groups, such as trimethylsilyl or t-butyl dimethylsilyl groups, or as tetrahydropyran derivatives.

Removal of any protecting groups present may be achieved by conventional procedures. Thus an aralkyl group such as benzyl, may be cleaved by hydrogenolysis in the presence of a catalyst (e.g. palladium on charcoal); an acyl group such as N-benzyloxycarbonyl may be removed by hydrolysis with, for example, hydrogen bromide in acetic acid or by reduction, for example by catalytic hydrogenation; silicon protecting groups may be removed, for example by treatment with fluoride ion or by hydrolysis under acidic conditions; tetrahydropyran groups may be cleaved by hydrolysis under acidic conditions.

As will be appreciated, in any of the general processes (A) to (D) described above it may be necessary or desired to protect any sensitive groups in the molecule as just described. Thus, a reaction step involving deprotection of a protected derivative of general formula (I) or a salt thereof may be carried out subsequent to any of the above described processes (A) to (D).

Thus, according to a further aspect of the invention, the following reactions may, if necessary and/or desired by carried out in any appropriate sequence subsequent to any of the processes (A) to (D)
(i) removal of any protecting groups; and
(ii) conversion of a compound of formula (I) or a salt thereof into a pharmaceutically acceptable salt thereof.

Where it is desired to isolate a compound of the invention as a salt, for example as an acid addition salt, this may be achieved by treating the free base of general formula (I) with an appropriate acid, preferably with an equivalent amount, or with creatinine sulphate in a suitable solvent (e.g. aqueous ethanol).

As well as being employed as the last main step in the preparative sequence, the general methods indicated above for the preparation of the compounds of the invention may also be used for the introduction of the desired groups at an intermediate stage in the preparation of the required compound. It should therefore be appreciated that in such multi-stage processes, the sequence of reactions should be chosen in order that the reaction conditions do not affect groups present in the molecule which are desired in the final product.

The biological activity of the compounds of formula (I) appears to reside in the S enantiomer, and the R enantiomer has little or no activity. Thus, the S enantiomer of a compound of formula (I) is generally preferred over a mixture of R and S such as the racemic mixture. However, if the R enantiomer were desired, *e.g*., for control studies or synthesis of other compounds, it could be conveniently prepared by the procedure above using the R enantiomer of the compound of formula (III) prepared according to the teachings of '512.

A compound of formula (I) prepared by reaction Scheme I, IA or 1B may be purified by conventional methods of the art, *e.g.*, chromatography, distillation or crystallization.

### Cleavable Complex in vitro Assay

The data in Table A, below, shows the relative topoisomerase Type I inhibitory activity of the compounds of Formula (I). This assay performed according to the method described in Hsiang, Y. et al., *J. Biol. Chem.*, 260:14873-14878 (1985), correlates well with *in vivo* anti-tumor activity of topoisomerase inhibitors in animal models of cancer, *e.g*., camptothecin and its analogs. See Hsiang et al., *Cancer Research,* 49:4385-4389 (1989) and Jaxel et al., *Cancer Research*, 49:1465-1469 (1989).

Those compounds which exhibit observable activity at concentrations greater than 2000 nM ("+" in Table A) are considered weakly to moderately active, while those with activity at concentrations less than 500 nM ("++++" in Table A) are very active. The term "IC₅₀" means the concentration of a compound of formula (I) at which 50% of the DNA substrate has been captured by topoisomerase I.

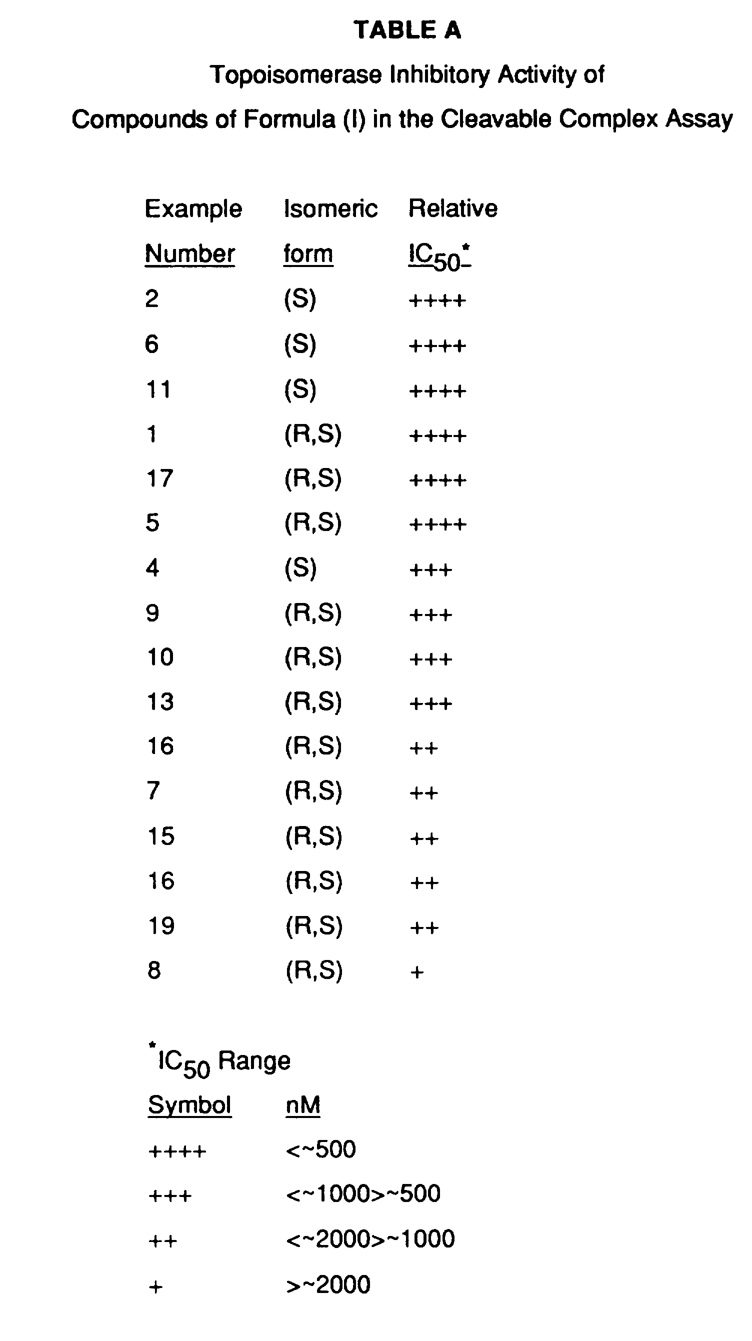

The compounds of formula (IV) have also been found to have good topoisomerase I inhibitory activity.

### Human Tumor Xenografts

In recent years, human tumor xenografts heterotransplanted into nude mice have been widely used to assess the antitumor activities of cancer chemotherapeutic agents. See Giovanella, B.C., Stehlin, Jr., J.S., Shepard, R.C. and Williams, Jr., L.J., "Correlation between response to chemotherapy of human tumors in patients and in nude mice", *Cancer* **52**:1146-1152, (1983); Boven, E. and Winograd, B, Eds. *The Nude Mouse in Oncology Research* CRC Press, Inc., Boca Raton, FL, (1991); and Fiebig, H.H., "Comparison of tumor response in nude mice and in patients", *Human Tumour Xenografts in Anticancer Drug Development*, Winograd, B., Peckham, M.J., and Pinedo, H.M., Eds., E.S.O. Mongraphs, Springer, Heidelberg, **25** (1988).

In general, human tumor xenografts retain not only the histological, biochemical and antigenic characteristics, but also the chemosensitivity of the tumor tissue of origin (Boven, *et al., supra*). Lengthy studies have provided evidence that human tumor xenografts retain these important biological properties of the tumor of origin including a biological instability as is known to occur in patient's tumors (Boven, *et al., supra*). Most importantly, several investigators have reported good correlations between drug effects in the human tumor xenografts and clinical results in human patients (Giovanella, *et al*., and Fiebig *supra*).

### Human Colorectal Adenocarcinoma HT-29 Xenograftin vivo Assay

Female NU/NU mice weighing 21 ± 2g, are used for this modified version of the test described by B.C. Giovanella, *et al., Science*, **246** 1046 (1989). Control and test animals are injected subcutaneously in the sub-scapular region with a suspension of 10⁶ viable HT-29 human colon tumor cells on day 0. Tumors are allowed to grow for 2 weeks prior to drug administration. For each drug, several doses are chosen based on its *in vitro* activity against topoisomerase I. Each dose level group contains 8 animals. The test compounds are prepared in either 0.1 M acetate buffer, pH 5 (vehicle "a") or 87.5% phosphate buffered saline, 12.375% dimethylsulfoxide, and 0.125% Tween 80 (trademark of ICI America for polyoxyethylenesorbitan monooleate) (vehicle "b") and are administered subcutaneously twice a week for 5 weeks beginning on day 14. Doses are given on a mg/kg basis according to the mean body weight for each cage.

Tumor weight is calculated from two perpendicular caliper measurements of the tumor using the formula, tumor weight = length x width ö 2 in millimeters. For each animal, tumor weight is monitored over the course of the experiment. For each group, the results are expressed as the ratio of the mean tumor weight immediately after 5 weeks of treatment (day 50) divided by the mean tumor weight immediately before treatment (day 14). Results are expressed in Table B. For either of the vehicle controls, the ratio is approximately 20, indicating that the tumor, in the absence of drug treatment, increased in weight approximately 20-fold over the course of the experiment. In contrast, a ratio of 1 indicates tumor stasis while a ratio less than 1 indicates tumor regression. Thus, compounds 4 and 6 caused tumor stasis while compounds 11 and 23 caused tumor regression. The criterion for antitumor activity is at least 50% inhibition of tumor growth after 5 weeks of dosing (day 50), giving a ratio of less than or equal to ten.

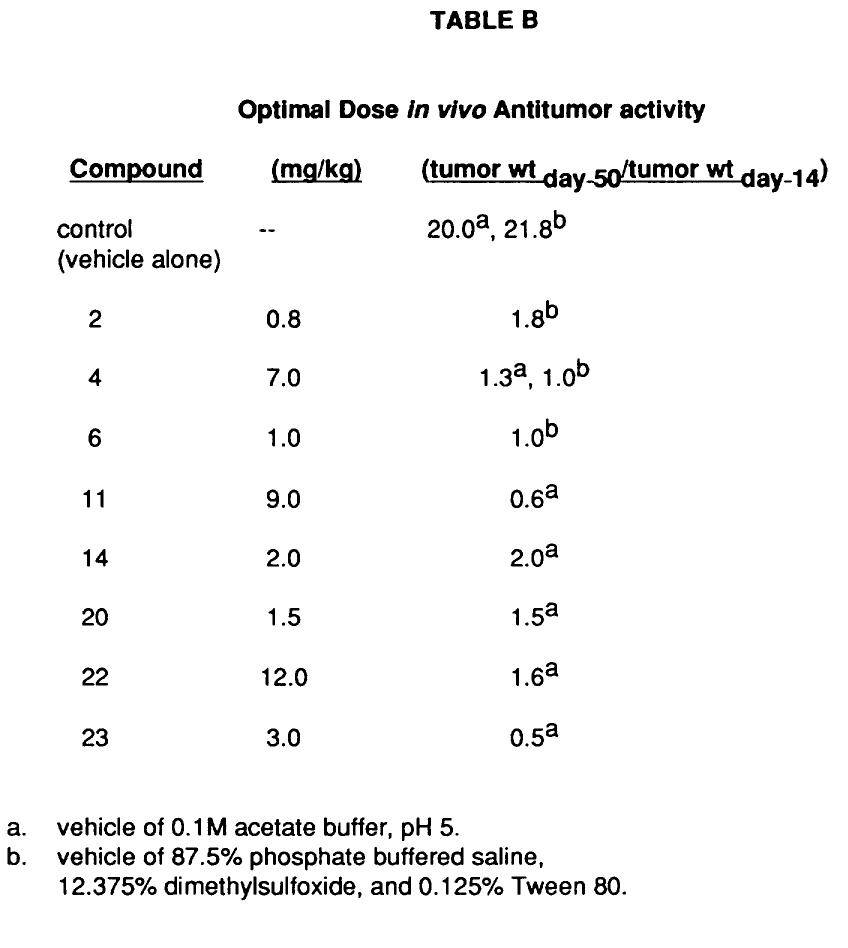

### Utility

In view of such activity, the compounds of formula (I) are active against a wide spectrum of mammalian (including human) tumors and cancerous growths such as cancers of the oral cavity and pharynx (lip, tongue, mouth, pharynx), esophagus, stomach, small intestine, large intestine, rectum, liver and biliary passages, pancreas, larynx, lung (including non-small cell lung carcinoma), bone, connective tissue, skin, colon, breast, cervix uteri, corpus endometrium, ovary, prostate, testis, bladder, kidney and other urinary tissues, eye, brain and central nervous system, thyroid and other endocrine gland, leukemias (lymphocytic, granulocytic, monocytic), Hodgkin's disease, non-Hodgkin's lymphomas, multiple myeloma, etc. Herein the terms "tumor", "cancer" and "cancerous growths" are used synonymously.

The amount of compound of formula (I) required to be effective as an antitumor agent will, of course, vary with the individual mammal being treated and is ultimately at the discretion of the medical or veterinary practitioner. The factors to be considered include the condition being treated, the route of administration, the nature of the formulation, the mammal's body weight, surface area, age and general condition, and the particular compound to be administered. However, a suitable effective antitumor dose is in the range of about 0.1 to about 200 mg/kg body weight per day, preferably in the range of about 1 to about 100 mg/kg per day. The total daily dose may be given as a single dose, multiple doses, e.g., two to six times per day, or by intravenous infusion for a selected duration. Dosages above or below the range cited above are within the scope of the present invention and may be administered to the individual patient if desired and necessary.

For example, for a 75 kg mammal, a dose range would be about 75 to about 7500 mg per day, and a typical dose would be about 800 mg per day. If discrete multiple doses are indicated, treatment might typically be 200 mg of a compound of formula (I) given 4 times per day.

### Formulations

Formulations of the present invention, for medical use, comprise an active compound, *i.e*., a compound of formula (I), together with an acceptable carrier thereof and optionally other therapeutically active ingredients. The carrier must be pharmaceutically acceptable in the sense of being compatible with the other ingredients of the formulation and not deleterious to the recipient thereof.

The present invention, therefore, further provides a pharmaceutical formulation comprising a compound of formula (I) together with a pharmaceutically acceptable carrier thereof.

The formulations include those suitable for oral, rectal, vaginal, transdermal or parenteral (including subcutaneous, intramuscular and intravenous) administration. Preferred are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier and then, if necessary, shaping the product into desired unit dosage form.

Formulations of the present invention suitable for oral administration may be presented as discrete units such as capsules, cachets, tablets or lozenges, each containing a predetermined amount of the active compound; as a powder or granules; or a suspension or solution in an aqueous liquid or non-aqueous liquid, *e.g.*, a syrup, an elixir, an emulsion or a draught.

A tablet may be made by compression or molding, optionally with one or more accessory ingredients. Compressed tablets may be prepared by compressing in a suitable machine the active compound in a free-flowing form, *e.g*., a powder or granules, optionally mixed with accessory ingredients, *e.g.*, binders, lubricants, inert diluents, surface active or dispersing agents. Molded tablets may be made by molding in a suitable machine, a mixture of the powdered active compound with any suitable carrier.

A syrup or suspension may be made by adding the active compound to a concentrated, aqueous solution of a sugar, *e.g.,* sucrose, to which may also be added any accessory ingredients. Such accessory ingredient(s) may include flavoring, an agent to retard crystallization of the sugar or an agent to increase the solubility of any other ingredient, *e.g*., as a polyhydric alcohol, for example, glycerol or sorbitol.

Formulations for rectal or vaginal administration may be presented as a suppository with a conventional carrier, *e.g*., cocoa butter or Witepsol S55 (trademark of Dynamite Nobel Chemical, Germany, for a suppository base).

For transdermal administration the compounds according to the invention may be formulated as creams, gels, ointments or lotions or as a transdermal patch. Such compositions may for example be formulated with an aqueous or oily base with the addition of suitable thickening, gelling, emulsifying, stabilising, dispersing, suspending, and/or colouring agents.

Formulations suitable for parenteral administration conveniently comprise a sterile aqueous preparation of the active compound which is preferably isotonic with the blood of the recipient. Such formulations suitably comprise a solution or suspension of a pharmaceutically and pharmacologically acceptable acid addition salt of a compound of the formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the hydrochloride. Useful formulations also comprise concentrated solutions or solids containing the compound of formula (I) which upon dilution with an appropriate solvent give a solution suitable for parenteral administration above.

In addition to the aforementioned ingredients, the formulations of this invention may further include one or more optional accessory ingredient(s) utilized in the art of pharmaceutical formulations, *e.g.,* diluents, buffers, flavoring agents, binders, surface active agents, thickeners, lubricants, suspending agents, preservatives (including antioxidants) and the like.

### EXAMPLES

The following examples illustrate aspects of this invention but should not be construed as limitations. The symbols and conventions used in these examples are consistent with those used in the contemporary chemical literature, for example, the *Journal of the American Chemical Society*. As used here in the term "room temperature" means about 25^{o}C.

### Example 1

### 7-Dimethylaminomethylene-10, 11-methylenedioxy-20(R,S)-camptothecin (Compound 1)

### (A) 3,4-Methylenedioxyacetanilide

To commercially available 3,4-methylenedioxy aniline (17.0g, 124 mmol)and sodium carbonate (15.5g, 136 mmol)in chloroform (90mL) at 5^{o}C is added acetyl chloride (8.8g, 124 mmol) dropwise with stirring. The reaction is allowed to warm to room temperature and stirring is continued for about 18 hours. The reaction mixture is washed twice with about 50 mL of 1N HCl and the organic layer is dried (MgSO₄) and the solvent removed to yield a brown solid. Recrystallization from water with activated carbon treatment yields 3,4-methylenedioxyacetanilide (9.34g, 42.1% of theory) as a light brown solid.

| Elemental analysis: (C₉H₉NO₃) | | | |
|---|---|---|---|
| | %C | %H | %N |
| Found: | 60.34 | 5.04 | 7.79 |
| Calculated | 60.33 | 5.06 | 7.82 |

### (B) 2'-Acetylamino-4',5'-methylenedioxy-2-chloroacetophenone

To a mixture of zinc chloride (24.3g, 178.3 mmol) and chloroacetylchloride (16.1 mL, 202.1 mmol) in nitromethane (85mL), under nitrogen, at room temperature, with stirring, is added, dropwise, 3,4-methylenedioxyacetanilide (8.96g, 50.0 mmol) in nitromethane (15mL). This mixture is then heated at reflux for 1.5 hrs, allowed to cool to room temperature, poured over ice, extracted with methylene chloride, which is then removed by evaporation, to yield a brown solid. This solid is recrystallized from an ethyl acetate / hexane mixture (including treatment with activated charcoal) to yield 2'-acetylamino-4',5'-methylenedioxy-2-chloroacetophenone (831.3mg, 6.5% of theory) as yellow crystals.
¹H-NMR (CDCl₃): δ 8.45 (s, 1H); 7.2 (s, 1H); 6.09 (s, 2H); 4.65 (s, 2H); 2.25 (s, 3H).

### (C) 3,4-Methylenedioxypivaloylanilide

This compound is prepared by the method of Example 1(a) except an equivalent amount of 2,2-dimethylpropanoyl chloride is used in place of acetyl chloride.

### (D) 2'-Pivoylamino-4',5'-methylenedioxy-2-chloroacetophenone

This compound is prepared by the method of Example 1(B) except an equivalent amount of 3,4-methylenedioxypivaloylanilide is used in place of 3,4-methylenedioxyacetanilide.

### (E) 2'-Amino-4',5'-methylenedioxy-2-chloroacetophenone

To 2'-acetylamino-4',5'-methylenedioxy-2-chloroacetophenone (0.9g, 3.53 mmol) or an equivalent amount of 2'-pivoylamino-4',5'-methylenedioxy-2-chloroacetophenone in ethanol (60mL) at about 5°C is added, dropwise, conc. HCI (12.5mL, 149.7 mmol). The reaction mixture is then heated at reflux for about an hour, then poured over 2N NaOH/ice (80mL60g) and washed with ethyl acetate (3 X 70mL). The organic portions are combined and washed with brine (50mL), dried (anhydrous sodium sulfate) and concentrated *in vacuo* to yield a greenishyellow solid. This solid is recrystallized from ethyl acetate/isopropanol/hexane, treated with activated charcoal, to yield 2'-amino-4',5'-methylenedioxy-2-chloroacetophenone (0.39g, 52% of theory).

| Elemental analysis: (C₉H₈NO₃Cl) | | | |
|---|---|---|---|
| | %C | %H | %N |
| Found: | 50.66 | 3.80 | 6.47 |
| Calculated | 50.60 | 3.77 | 6.56 |

### (F) 5'(R,S)-1,5-Dioxo-(5'-ethyl-5'-hydroxy-2'H,5'H,6'H-6-oxopyrano)[3', 4'-f]Δ⁶ʼ⁸-tetrahydroindolizine and 5'(S)-1,5-Dioxo-(5'-ethyl-5'-hydroxy-2'H,5'H,6'H-6-oxo-pyrano)[3', 4'-f]Δ⁶ʼ⁸-tetrahydroindolizine (compounds of formula (III))

These compounds, referred to hereinafter as "tricyclic ketone (R,S)" and "tricyclic ketone (S)" respectively or collectively as "a compound of formula (III)", are prepared according to the procedure taught by Wani *et al.,* in '512. Note that the corresponding R enantiomer may also be prepared by the procedure of '512.

### (G) 7-Chloromethyl-10,11-methylenedioxy-20(R,S)-camptothecin

Following the general procedure for camptothecin taught in '512, 2'-amino-4',5'-methylenedioxy-2-chloroacetophenone is stirred in refluxing toluene (50 mL) with tricyclic ketone (R,S) (256.3mg, 0.97 mmol) under a Dean-Stark trap for half an hour. The reaction is then cooled and the solid filtered and washed with toluene and ethanol to yield 7-chloromethyl-10,11-methylenedioxy-20(R,S)-camptothecin, (408.5mg, 68.8%).
¹H-300 NMR (DMSO-d6): δ 7.72 (s, 1H); 7.55 (s, 1H); 7.2 (s, 1H); 6.34 (s, 2H); 5.42 (s, 2H); 5.32 (s, 2H); 5.24 (s, 2H);1.85 (m, 2H); 0.88 (t, 3H).

| Nominal Mass Spectrum M+1: | |
|---|---|
| Calcd.: | 441 |
| Found: | 441 |

### (H) 7-Dimethylaminomethylene-10, 11-methylenedioxy-20(H,S)-camptothecin

To a stirred mixture of 7-chloromethyl-10,11-methylenedioxy-(R,S)-camptothecin (0.11g, 0.25 mmol) and potassium carbonate (346mg, 0.5 mmol) in dimethylformamide (DMF) (1mL) is added dimethylamine (6.1 mL, 0.5 mmol) in the form of a 3.73 mg/mL solution in tetrahydrofuran at about 5°C. The reaction mixture is securely stoppered, allowed to warm to room temperature, stirred for about 15 hrs and then filtered to remove the solid material. The filtrate is concentrated by vacuum evaporation and the resulting solid triturated with acetonitrile and filtered. The filtrate is concentrated by vacuum evaporation to a thick residue. The residue is dissolved in minimal amount of chloroform and chromatographed on 30 grams of flash grade silica gel eluting with successive portions of 250 mls of ethyl acetate followed by 250 mls of (9:1 ethyl acetate, isopropanol finally with 250 mls of (4:1 ethyl acetate, isopropanol). Fractions were collected and monitored by TLC (5% methanol, ethyl acetate) and visualized by a UV lamp. The appropriate fractions were pooled, concentrated and dried under vacuo to yield 7-dimethylaminomethylene-10, 11-methylenedioxy-20(R,S)-camptothecin (6.0mg, 4.7%). This compound was characterized as its acetic acid salt.
m.p. >250°C

| Elemental analysis: (C₂₄H₂₃N₃O₃·C₂H₄O₂) | | | |
|---|---|---|---|
| | %C | %H | %N |
| Found: | 61.64 | 5.17 | 8.73 |
| Calculated | 61.29 | 5.34 | 8.25 |

### (I) Open E ring form

The compound of part (H) is treated with an equivalent amount of sodium hydroxide to form the corresponding open E ring form. Treatment of the latter with an equivalent amount of hydrochloric acid closes the E ring and thereby reforms the compound of part (H).

### Example 2

### 7-Dimethylaminomethylene-10, 11 -methylenedioxy-20(S)-camptothecin (Compound 2)

### (A) 7-Chloromethyl-10,11-methylenedioxy-20(S)-camptothecin

This compound is prepared by the procedure of Example 1, except in part (G) an equivalent amount of tricyclic ketone (S) is used in place of tricyclic ketone (R,S).
m.p. >250°C

### (B) 7-Dimethylaminomethylene-10,11-methylenedioxy-20(S)-camptothecin

This compound is prepared by the procedure of Example 1, part (H), except that an equivalent amount of 7-chloromethyl-10,11-methylenedioxy-20(S)-camptothecin, prepared according Example 2, part (A), is used in place of 7-chloromethyl-10,11-methylenedioxy-20(R,S)-camptothecin.
m.p. >250°C

| Nominal Mass Spectrum M+1: | |
|---|---|
| Calcd.: | 450 |
| Found: | 450 |

### Example 3

### 7-Dimethylaminomethylene-10, 11-ethylenedioxy-20(R,S)-camptothecin (Compound 3)

### (A) 7-Chloromethyl-10,11-ethylenedioxy-20(R,S)-camptothecin

This compound is prepared by the procedure of Example 1, except in parts (A) and (C) an equivalent amount of 3,4-ethylenedioxy aniline is used in place of 3,4-methylenedioxy aniline.

| High Resolution Mass Spectrum M+1: | |
|---|---|
| Calcd.: | 455.1009 |
| Found: | 455.1005 |

### (B) 7-Dimethylaminomethylene-10,11-ethylenedioxy-20(R,S)-camptothecin

This compound is prepared by the procedure of Example 1, part (H), except that an equivalent amount of 7-chloromethyl-10,11-ethylenedioxy-20(R,S)-camptothecin is used in place of 7-chloromethyl-10,11-methylenedioxy-20(R,S)-camptothecin.

| High Resolution Mass Spectrum: | |
|---|---|
| Calcd.: | 464.1821 |
| Found: | 464.1833 |

### Example 4

### 7-Dimethylaminomethylene-10,11-ethylenedioxy-20(S)-camptothecin (Compound 4)

### (A) 7-Chloromethyl-10,11-ethylenedioxy-20(S)-camptothecin

This compound is prepared by the procedure of Example 1 except in parts (A) and (C) an equivalent amount of 3,4-ethylenedioxy aniline is used in place of 3,4-methylenedioxy aniline, and in part (G) an equivalent amount of tricyclic ketone (S) is used in place of tricyclic ketone (R,S)

| High Resolution Mass Spectrum M+1: | |
|---|---|
| Calcd.: | 455.1009 |
| Found: | 455.1000 |

### (B) 7-Dimethylaminomethylene-10,11-ethylenedioxy-20(S)camptothecin

This compound is prepared by the procedure of Example 1, part (H), except that an equivalent amount of 7-chloromethyl-10,11-ethylenedioxy-20(S)-camptothecin in place of 7-chloromethyl-10,11-methylenedioxy-20(R,S)-camptothecin.

| High Resolution Mass Spectrum: | |
|---|---|
| Calcd.: | 464.1821 |
| Found: | 464.1811 |

### Example 5

### 7-Morpholinomethylene-10,11-ethylenedioxy-20(R,S)-camptothecin(Compound 5)

The same procedure as Example 1, part (H), is used except that an equivalent amount of morpholine is used in place of dimethylamine and an equivalent amount of 7-chloromethyl-10,11-ethylenedioxy-20(H,S)-camptothecin, prepared according to Example 3, part (B), is used in place of 7-chloromethyl-10,11 -methylenedioxy-20(R,S)-camptothecin.

| High Resolution Mass Spectrum: | |
|---|---|
| Calcd.: | 506.1942 |
| Found: | 506.1925 |

### Example 6

### 7-Morpholinomethylene-10,11-ethylenedioxy-20(S)-camptothecin (Compound 6)

The same procedure as Example 1, part (H), is used except that an equivalent amount of morpholine is used in place of dimethylamine and an equivalent amount of 7-chloromethyl-10,11-ethylenedioxy-20(S)-camptothecin, prepared according to Example 4, part (B), is used in place of 7-chloromethyl-10,11-methylenedioxy-20(R,S)-camptothecin.

| High Resolution Mass Spectrum: | |
|---|---|
| Calcd.: | 506.1942 |
| Found: | 506.1929 |

### Example 7

### 7-Pyrrolidinomethylene-10,11-ethylenedioxy-20(R,S)-camptothecin (Compound 7)

The same procedure as Example 1, part (H), is used except that an equivalent amount of pyrrolidine is used in place of dimethylamine and an equivalent amount of 7-chloromethyl-10,11-ethylenedioxy-20(R,S)-camptothecin, prepared according to Example 3, part (A), is used in place of 7-chloromethyl-10,11-methylenedioxy-20(R,S)-camptothecin.

| High Resolution Mass Spectrum: | |
|---|---|
| Calcd.: | 490.1978 |
| Found: | 490.1988 |

### Example 8

### 7-Piperidinomethylene-10,11-methylenedioxy-20(R,S)-camptothecin (Compound 8)

The same procedure as Example 1, part (H), is used except that an equivalent amount of piperidine is used in place of dimethylamine.

| Nominal Mass Spectrum M+1: | |
|---|---|
| Calcd.: | 490 |
| Found: | 490 |

¹H-300 NMR (DMSO-d6): δ 7.95(s,1H); 7.62(s, 1H); 7.29(s, 1H); 6.35(s, 2H); 5.49(s, 2H); 5.41(s, 2H); 4.85(broad s, 2H); 1.9 - 0.7 (m, 11H).

### Example 9

### 7-Piperidinomethylene-10,11-ethylenedioxy-20(R,S)-camptothecin (Compound 9)

The same procedure as Example 1, part (H), is used except that an equivalent amount of piperidine is used in place of dimethylamine and an equivalent amount of 7-chloromethyl-10,11-ethylenedioxy-20(R,S)-camptothecin, prepared according to Example 3, part (A), is used in place of 7-chloromethyl-l0,11-methylenedioxy-20(R,S)-camptothecin.

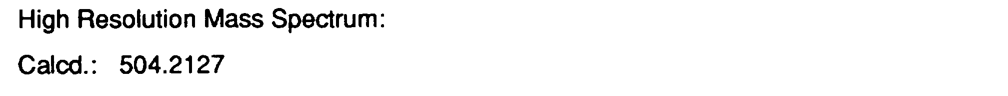

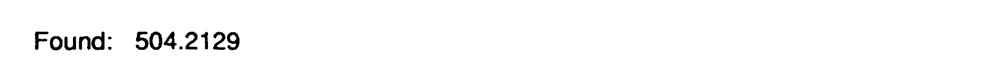

### Example 10

### 7-(4-methylpiperazinomethylene)-10,11-ethylenedioxy-20(R,S)-camptothecin (Compound 10)

The same procedure as Example 1, part (H), is used except that an equivalent amount of 4-methylpiperazine is used in place of dimethylamine and an equivalent amount of 7-chloromethyl-10,11-ethylenedioxy-20(R,S)-camptothecin, prepared according to Example 3, part (A), is used in place of 7-chloromethyl-10,11-methylenedioxy-20(R,S)-camptothecin.

| High Resolution Mass Spectrum : | |
|---|---|
| Calcd.: | 519.2236 |
| Found: | 519.2246 |

### Example 11

### 7-(4-methylpiperazinomethylene)-10,11-ethylenedioxy-20(S)-camptothecin (Compound 11)

The same procedure as Example 1, part (H), is used except that an equivalent amount of 4-methylpiperazine is used in place of dimethylamine and an equivalent amount of 7-chloromethyl-10,11 -ethylenedioxy-20(S)-camptothecin, prepared according to Example 4, part (A), is used in place of 7-chloromethyl-10,11 -methylenedioxy-20(R,S)-camptothecin.
m.p. 261-264°C

| Nominal Mass Spectrum M+1: | |
|---|---|
| Calcd.: | 519 |
| Found: | 519 |

### Example 12

### 7-Diethylaminomethylene-10,11-methylenedioxy-20(S)-camptothecin (Compound 12)

This compound is prepared by the procedure of Example 1, part (H), except that equivalent amount of diethylamine is used in place of dimethylamine and an equivalent amount of 7-chloromethyl-10,11-methylenedioxy-20(S)-camptothecin, prepared according Example 2, part (A), is used in place of 7-chloromethyl-10,11-methylenedioxy-20(R,S)-camptothecin.

| High Resolution Mass Spectrum: | |
|---|---|
| Calcd. | 478.1978 |
| Found: | 478.1963 |

### Example 13

### 7-Diethylaminomethylene-10,11-ethylenedioxy-20(R,S)-camptothecin (Compound 13)

The same procedure as Example 1, part (H), is used except that an equivalent amount of diethylamine is used in place of dimethylamine and an equivalent amount of 7-chloromethyl-10,11-ethylenedioxy-20(R,S)-camptothecin, prepared according to Example 3, part (A), is used in place of 7-chloromethyl-l0,11-methylenedioxy-20(R,S)-camptothecin.

| High Resolution Mass Spectrum: | |
|---|---|
| Calcd.: | 492.2134 |
| Found: | 492.2140 |

### Example 14

### 7-Diethylaminomethylene-10,11-ethylenedioxy-20(S)-camptothecin (Compound 14)

The same procedure as Example 1, part (H), is used except that an equivalent amount of diethylamine is used in place of dimethylamine and an equivalent amount of 7-chloromethyl-10,11-ethylenedioxy-20(S)-camptothecin, prepared according to Example 4, part (A), is used in place of 7-chloromethyl-10,11-methylenedioxy-20(R,S)-camptothecin.

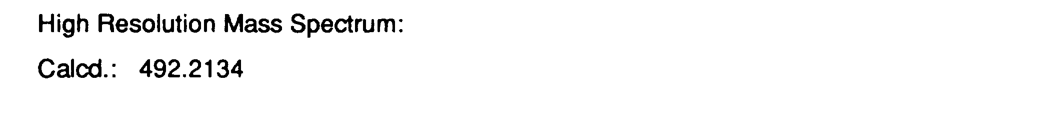

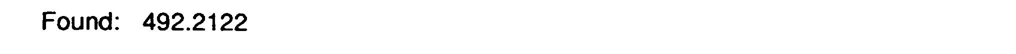

### Example 15

### 7-N-Methylethanolaminomethylene-10,11-methylenedioxy-20(R,S)-camptothecin (Compound 15)

The same procedure as Example 1, part (H), is used except that an equivalent amount of N-methylethanolamine is used in place of dimethylamine.

| High Resolution Mass Spectrum: | |
|---|---|
| Calcd.: | 480.1771 |
| Found: | 480.1776 |

### Example 16

### 7-N-Methylethanolaminomethylene-10,11-ethylenedioxy-20(R,S)-camptothecin (Compound 16)

The same procedure as Example 1, part (H), is used except that an equivalent amount of N-methylethanolamine is used in place of dimethylamine and an equivalent amount of 7-chloromethyl-10,11-ethylenedioxy-20(H,S)-camptothecin, prepared according to Example 3, part (A), is used in place of 7-chloromethyl-10,11-methylenedioxy-20(R,S)-camptothecin.

| High Resolution Mass Spectrum: | |
|---|---|
| Calcd.: | 494.1927 |
| Found: | 494.1929 |

### Example 17

### 7-Diethanolaminomethylene-10,11-ethylenedioxy-20(R,S)-camptothecin (Compound 17)

The same procedure as Example 1, part (H), is used except that an equivalent amount of diethanolamine is used in place of dimethylamine and an equivalent amount of 7-chloromethyl-10,11-ethylenedioxy-20(R,S)-camptothecin, prepared according to Example 3, part (A), is used in place of 7-chloromethyl-10,11-methylenedioxy-20(R,S)-camptothecin.

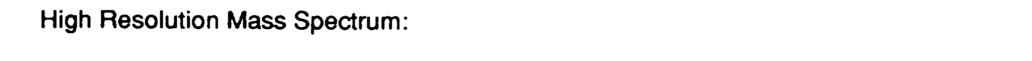

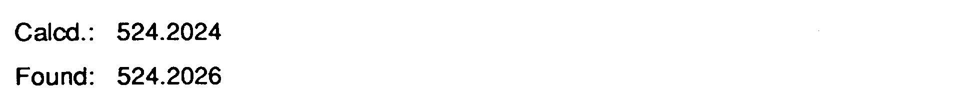

### Example 18

### 7-Diethanolaminomethylene-10-11-ethylenedioxy-20(S)-camptothecin (Compound 19)

The same procedure as Example 1, part (H), is used except that an equivalent amount of diethanolamine is used in place of dimethylamine and an equivalent amount of 7-chloromethyl-10,11-ethylenedioxy-20(S)-camptothecin, prepared according to Example 4, part (A), is used in place of 7-chloromethyl-10,11-methylenedioxy-20(R,S)-camptothecin.
m.p. 230-233°C.

| Nominal Mass Spectrum M+1: | |
|---|---|
| Calcd.: | 524 |
| Found: | 524 |

### Example 19

### 7-Azetidinomethylene-10,11-methylenedioxy-20(R,S)-camptothecin (Compound 19)

The same procedure as Example 1, part (H), is used except that an equivalent amount of azetidine is used in place of dimethylamine. m.p. >250°C.

| Nominal Mass Spectrum M+1: | |
|---|---|
| Calcd.: | 462 |
| Found: | 462 |

### Example 20

### 7-Azetidinomethylene-10, 11-methylenedioxy-20(S)camptothecin (Compound 20)

This compound is prepared by the procedure of Example 1 except in parts (G) an equivalent amount of tricyclic ketone (S) is used in place of tricyclic ketone (R,S), and in part (H) and equivalent amount of azetidine is used in place of dimethylamine.

| High Resolution Mass Spectrum: | |
|---|---|
| Calcd.: | 462.1665 |
| Found: | 462.1667 |

### Example 21

### 7-Thiomorpholinomethylene-10, 11-ethylenedioxy-20(S)-camptothecin (Compound 21)

The same procedure as Example 1, part (H), is used except that an equivalent amount of thiomorpholine is used in place of dimethylamine and an equivalent amount of 7-chloromethyl-10,11-ethylenedioxy-20(S)-camptothecin, prepared according to Example 4, part (A), is used in place of 7-chloromethyl-l0,11-methylenedioxy-20(R,S)-camptothecin.
m.p. 249-252°C.

| Nominal Mass Spectrum M+1: | |
|---|---|
| Calcd.: | 522 |
| Found: | 522 |

### Example 22

### 7-Azetidinomethylene-10, 11-ethylenedioxy-20(S)-camptothecin (Compound 22)

The same procedure as Example 1, part (H), is used except that an equivalent amount of azetidine is used in place of dimethylamine and an equivalent amount of 7-chloromethyl-10,11-ethylenedioxy-20(S)-camptothecin, prepared according to Example 4, part (A), is used in place of 7-chloromethyl-10,11-methylenedioxy-20(R,S)-camptothecin.
m.p. 208-210 (decomp.) Low Resolution Mass Spectrum: 476.2 (electron spray).

### Example 23

### 7-(4-Methylpiperazinomethylene)-10, 11-methylenedioxy-20(S)-camptothecin (Compound 23)

This compound is prepared by the procedure of Example 1 except in parts (G) an equivalent amount of tricyclic ketone (S) is used in place of tricyclic ketone (R,S), and in part (H) and equivalent amount of 4-methylpiperazine is used in place of dimethylamine.

| High Resolution Mass Spectrum: | |
|---|---|
| Calcd.: | 505.2083 |
| Found: | 505.2087 |

### Example 24

### 7-Trifluoroacetamidomethylene-10, 11-ethylenedioxy-20(S)-camptothecin (Compound 24)

### (A) 2'-Amino-4',5'-methylenedioxy-2-trifluoroacetamidoacetophenone

Trifluoroacetamine (227mg, 2 mmole) is added to a solution of cesium carbonate (1.63g, 5 mmole) in anhydrous acetonitrile (15ml) at room temperature under nitrogen. 2'-Amino-4',5'-methylenedioxy-2-chloroacetophenone is then added and the mixture is placed in a preheated oil bath set at 90°C for 30 minutes. The reaction is cooled to room temperature and poured directly onto a silica plug (15g) in a scintered glass funnel. The silica is washed two times with EtOAc and the volatiles from the combined washes are removed in vacuo. Diethyl either is used to triturate the residue to afford a light orange solid which is collected by filtration and dried under vacuum. (498 mg, 86%). Mp=219-220°C. ¹H NMR (300 MHz, DMSO-d⁶): δ 4.44 (d, 2H); 5.96 (s, 2H); 5,96 (s, 2H); 6.35 (s, 1H); 7.21 (s, 1H); 7.40 (bs, 2H); 9.59 (t, 1H). Nominal mass expected: MH+ = 291 m/z. found MH+ = 291 m/z.

### (B) 2'-Amino-4',5,-ethylenedioxy-2-trifluoroacetamidoacetophenone

This compound is prepared as in the method above except an equivalent amount of 2'-amino-4',5'-ethylenedioxy-2-chloroacetophenone is used in place of 2'-amino-4',5'-methylenedioxy-2-chloroacetophenone. A green solid is isolated in 74% yield. Mp = 154-155°C. ¹H NMR (300 MHz, CDCl₃): δ 4.08 (m, 2H); 4.13 (m, 2H); 4.60 (d, 2H); 6.0 (bs, 2H); 6.08 (s, 1H); 7.04 (s, 1H); 7.60 (t, 1H). Nominal mass expected: MH+ = 305 m/z. Found: MH+ = 305 m/z.

### (C) 10,11-Ethylenedioxy-7-trifluoroacetamidomethylene-20(S)-camptothecin

2'-Amino-4',5'-ethylenedioxy-2-trifluoroacetamidoacetophenone (71 mg, 0.234 mmole), tricyclic ketone (S) (61 mg, 0.234 mmole), and anhydrous toluene (2.0 ml) are combined at 60°C under nitrogen. A catlytic amount of both glacial acetic acid and p-toluenesulfonic acid monohydrate are added before increasing the reaction temperature to reflux. The reaction refluxes for 16 hrs and is then cooled to ambient temperature. A green-yellow solid is collected by filtration, washed with ethanol and diethyl ether, and dried in vacuo. (101 mg, 84%) Mp = 249°C. ¹H NMR (300 MHz, DMSO-d6): δ 0.91 (t, 3H); 1.91 (m, 2H); 4.40 (s, 4H); 4.83 (d, 2H); 5.39 (s, 2H); 5.41 (s, 2H); 6.48 (s, 1H); 7.22 (s, 1H); 7.58 (s, 1H); 7.77 (s, 1H); 10.20 (t, 1H). Nominal mass expected: MH+ = 532 m/z. Found: MH+ = 532 m/z.

### Example 25

### 7-Trifluoroacetamidomethylene-10,11-methylenedioxy-20(S)-camptothecin (Compound 25)

This compound is prepared by the method of Example 24 above except an equivalent amount of 2'-amino-4',5'-methylenedioxy-2-trifluoroacetamidoacetophenone is used in place of 2'-amino-4',5'-ethylenedioxy-2-trifluoroacetamidoacetophenone. A green-yellow solid is isolated in 15% yield mp = 238°C (d). ¹H-300 NMR (DMSO-d6): δ 0.91 (t, 3H); 1.95 (m, 2H); 4.92 (s, 2H); 5.38 (s, 2H); 5.40 (s, 2H); 6.28 (S, 2H); 6.49 (s, 1H); 7.13 (s, 1H); 7.58 (s, 1H); 7.78 (s, 1H); 10.21 (t, 1H). Nominal mass expected: MH+ = 518 m/z. Found MH+ = 518 m/z.

### Example 26

### 7-Aminomethylene-10,11-ethylenedioxy-20(S)-camptothecin dihydrochloride (Compound 26)

7-trifluoroacetamidomethylene-10,11-ethylenedioxy-20(S)-camptothecin (65 mg, 0.12 mmole) is heated to 105°C in aqueous 2N hydrochloric acid (1.2 ml) for 20 minutes in an open flask. the volatiles are removed in vacuo and the residue is triturated with ethyl acetate and collected by filtration. The bright yellow solid is washed with ethyl acetate (3 ml), ethanol (2 ml) and diethyl ether (2 ml) and dried in vacuo to afford 62 mg (100%). mp > 300°C. ¹H NMR (300 MHz, DMSO-d6): δ 0.90 (t, 3H); 1.95 (m, 2H); 4.41 (s, 4H); 4.61 (d, 2H); 5.40 (s, 2H); 5.45 (s, 2H); 7.24 (s, 1H); 7.60 (s, 1H); 7.81 (s, 1H); 8.40 (bs, 2H). Nominal mass expected: MH+ = 436 m/z. Found MH+ = 436 m/z.

### Example 27

### 7-Aminomethylene-10,11-methylenedioxy-20(S)-camptothecin dihydrochloride (Compound 27)

This compound is prepared by the method of Example 26 above except an equivalent amount of 7-trifluoroacetamidomethylene-10,11-methylenedioxy-20(S)-camptothecin is used in place of 7-trifluoroacetamidomethylene-10,11-ethylenedioxy-20(S)-camptothecin. A golden yellow solid is isolated in quantitative yield. Mp = 270°C (d). ¹H NMR (300 MHz, DMSO-d6): δ 0.90. (t, 3H); 1.9 (m, 2H); 4.6 (m, 2H); 5.4 (s, 2H); 5.5 (s, 2H); 6.3 (s, 2H); 7.2 (s, 1H); 7.6 (s, 1H); 7.9 (s, 1H); 8.4 (bs, 2H). Nominal mass expected: MH+ = 422 m/z. Found: MH+ = 422 m/z.

### Example 28

### 7-tert-Butyloxycarbonyl-piperazinomethylene 10,11-ethylenedioxy-20(S)-camptothecin (Compound 28)

To a -50°C solution of (S)-(-)-10,11-ethylenedioxy-7-chloromethyl-camptothecin (35.8 mg, 78.7 x 10⁻³ mmol) was added dropwise tert-butyl-l-piperazinecarboxylate (34.6 mg, 186 x 10⁻³ mmol) in N,N-dimethylformamide (DMF) (0.45 mL). The dark brown reaction mixture was stirred at -50°C for 10 min, and allowed to warm to 0°C. Additional tert-butyl-l-piperazinecarboxylate (8 mg, 43 X 10⁻³ mmol) in DMF (0.2 mL) was added, and the mixture was allowed to warm to ambient temperature. The mixture was stirred for an additional 90 min, and the solvent was removed with a rotary evaporator to afford the crude product as a brown residue. Purification by silica gel chromatography (eluting with 100% ethyl acetate) attorded 20.7 mg (58% yield) of the product as a pale yellow solid. ¹H NMR (300 MHz, CDCl₃): δ 1.04 (t, 3H, J=7); 1.45 (s, 9H); 1.87 (m, 2H); 2,46 (s, 4H); 3.41 (s, 4H); 3.94 (s, 2H); 4.43 (s, 4H); 5.29 (s, 2H); 5.30 (d, 1H, J=16); 5.75 (d, 1H, J=16); 7.59 (s, 1H); 7.65 (s, 1H); 7.73 (s, 1H).
Nominal mass spectrum (M+1): 605.

### Example 29

### 7-Piperazinomethylene-10,11-ethylenedioxy-20(S)-camptothecin trifluoroacetic acid salt (Compound 29)

To a 0°C solution of 7-*tert*-butyloxycarbonyl-piperazinomethyl-10,11-ethylenedioxy-20(S)-camptothecin (16.7 mg, 27.6 x 10⁻³ mmol) in dry CH₂Cl₂ (5.0 mL) was added trifluoroacetic acid (0.5 mL). The deep yellow solution was allowed to warm to ambient temperature and stirred for 14h. The mixture was concentrated with a rotary evaporator, and the residue was purified by reverse phase HPLC (Rainin Dynamax 60A column, eluting with 49:10:2.5:1 water/acetonitrile/THF/trifluoroacetic acid) to afford, after concentration and lyophylization of the major UV active peak (monitoring at 254 nm), 21.7 mg of the product as a yellow fluffy powder. ¹H NMR (300 MHz, DMSO-d₆): δ 0.88 (t, 3H, J=7); 1.87 (m, 2H); 2.60-2.80 (m, 4H); 3.00-3.20 (bs, 4H); 5.29 (s, 2H); 5.41 (s, 2H); 6.5 (bs, 1H); 7.25 (s, 1H); 7.56 (s, 1H); 7.80 (s, 1H); 8.50 (bs, 2H).
Nominal mass spectrum (M+1): 505.
mp: 315°C(d)

### Example 30

### 7-(α,α,α-Trifluoro-m-tolyl)-piperazinomethylene-10,11-ethylenedioxy-20(S)-camptothecin (Compound 30)

A solution of 7-chloromethyl-10,11-ethylenedioxy-20(S)-camptothecin (5.2 mg, 11.4 x 10⁻³ mmol) in anhydrous DMSO (200 µl) was added dropwise to a 0°C solution of 1-(α,α,α-trifluoro-m-tolyl)-piperazine (10 µl, 53 x 10⁻³ mmol) in anhydrous toluene (500 µl). The dark brown mixture was stirred at 0°C for 90 min, and allowed to warm to ambient temperature. The solvent was removed with a rotary evaporator and further pumping under high vacuum to leave the crude product, which was purified by silica gel chromatography (eluting wih 100% ethyl acetate followed by 6:5:1 ethyl acetate/chloroform/methanol) to afford 3.7 mg (50% yield) of the product as a pale yellow solid residue. ¹H NMR (200 MHz, DMSO-d₆): δ 0.90 (t, 3H, J=7); 1.95 (q, 2H, J=7); 2.60-2.70 (m, 4H); 3.20-3.30 (m, 4H); 4.10 (s, 2H); 4.50 (s, 4H); 5.30; (s, 2H); 5.45 (s, 2H); 6.55 (s, 1H); 7.40 (t, 1H, J=7); 7.60 (s, 1H); 7.85 (s, 1H).
Nominal mass spectrum (M+1): 649.

### Example 31

### 7-(2-Methoxyphenyl-piperazino)methylene-10,11-ethylenedioxy-20(S)-camtothecin (Compound 31)

To a 0°C solution of 2-methoxyphenylpiperazine (17.9 µl, 102 x 10⁻³ mmol) in anhydrous toluene (1 mL) at 0°C was added a solution of 7-chloromethyl-10,11-ethylenedioxy-20(S)-camptothecin (10 mg, 22 x 10⁻³ mmol) in DMSO (200 µl). The dark mixture was stirred at 0°C for 10 min, and allowed to warm to ambient temperature and stirred for 3h. The reaction mixture was concentrated with a rotary evaporator and the residual solvent was removed by pumping under high vacuum to afford the crude product. Purificaton by silica gel chromatography (eluting with 1:1 hexane/ethylacetate followed by 6:5:1 ethylacetate/chloroform/methanol) afforded 3.4 mg (25% yield) of the product as a yellow solid. ¹H NMR (200 MHz, CDCl₃): δ 1.05 (t, 3H, J=7); 1.90 (m, 2H); 2.75 (bs, 4H), 3.10 (bs, 4H), 3.75 (s, 1H); 3.85 (s, 3H); 4.01 (bs, 2H); 5.35 (s, 1H); 5.30 (d, 1H, J=18); 5.35 (s, 1 H); 5.75 (d, 1, J=18); 6.80-7.00 (m, 4H); 7.60 (s, 1H); 7.65 (s, 1H); 7.75 (s, 1H).

| Nominal mass spectrum (M+1): | |
|---|---|
| Calcd.: | 611 |
| Found: | 611. |

### Example 32

### 7-Phenylpiperazinomethylene-10,11-ethylenedioxy-20(S)-camptothecin (Compound 32)

To a 0°C solution of phenylpiperazine (15.6 µl, 102 x 10⁻³ mmol) in anhydrous toluene (1 mL) was added a solution of 7-chloromethyl-10,11-ethylenedioxy-20(S)-camptothecin (10.6 mg, 22 x 10⁻³ mmol) in DMSO (300 µl). The dark mixture was stirred at 0°C for 10 min, and allowed to warm to ambient temperature and stirred for 3h. The mixture was concentrated with a rotary evaporator, and the residual solvent was further removed by pumping under high vacuum to afford the crude product as a dark residue. Purification by silica gel chromatography (eluting with 1:1 hexane/ethyl acetate followed by 6:5:1 ethyl acetate/chloroform/methanol) afforded 3.6 mg (30% yield) of the product as a yellow solid residue. ¹H NMR (200 MHz, CDCl₃): δ 1.00 (t, 3H, J=7); 1.90 (m, 2H); 2.75 (bs, 4H); 3.20 (bs, 4H); 3.75 (s, 1H); 4.05 (s, 2H); 4.45 (bs, 4H); 5.35 (s, 2H); 5.30 (d, 1H, J=18); 5.35 (s, 2H); 5.75 (d, 1H); 6.80-7.00 (m, 3H); 7.20-7.35 (m, 2H); 7.60 (s, 1H); 7.65 (s, 1H); 7.80 (s, 1H).
Nominal mass spectrum (M+1): 581.

### Example 33

### 2'-Amino-4'5'-methylenedioxy-2-dimethylaminocetophenone

2'-Acetylamino-4',5'-methylenedioxy-2-chloroacetophenone, prepared in Example 1, part (B), is reacted with an excess of dimethylamine under similar conditions as taught in Example 1, part (H), to yield 2'-acetylamino-4',5'-methylenedioxy-2-dimethylaminoacetophenone which is turn is deprotected by the procedure of Example 1, part (E), to yield 2'-amino-4',5'-methylenedioxy-2-dimethylaminoacetophenone.

| Nominal Mass Spectrum M+1: | |
|---|---|
| Calcd.: | 223 |
| Found: | 223 |

### Examples 34 - 38

The following compounds of formula (I) are prepared by the procedure taught in Scheme I, IA or 1B in an analogous manner to Examples 1 - 32, using the appropriate intermediate compounds of formulas (II), (III), (IV) and (V).
34 7-(Methyl-2-methoxyethylaminomethylene)- 10, 11-methylenedioxy-20(R,S)-camptothecin,
35 7-Cyclohexylaminomethylene-10,11-methylenedioxy-20(R)-camptothecin,
36 7-(2-Butenyl)aminomethylene-10,11-methylenedioxy-20(R,S)-camptothecin,
37 7-Cyclohexylmethylaminomethylene-10,11-ethylenedioxy-20(R)-camptothecin and
38 7-Thiazolidinomethylene-10,11-methylenedioxy-20(R,S)-camptothecin.

### Example 39

### Pharmaceutical formulations

### (A) Transdermal System

| Ingredients | Amount |
|---|---|
| Active compound | 600.0 mg |
| Silicone fluid | 450.0 mg |
| Colloidal silicone dioxide | 25.0 mg |

The silicone fluid and active compound are mixed together and the colloidal silicone dioxide is reacted with to increase viscosity. The material is then dosed into a subsequently heat sealed polymeric laminate comprised of the following: polyester release liner, skin contact adhesive composed of silicone or acrylic polymers, a control membrane which is a polyolefin (e.g. polyethylene),polyvinyl acetate or polyurethane, and an impermeable backing membrane made of a polyester multilaminate. The system described is a 10 sq. cm patch.

### (B) Oral Tablet

| Ingredients | Amount |
|---|---|
| Active compound | 200.0 mg |
| Starch | 20.0 mg |
| Magnesium Stearate | 1.0 mg |

The active compound and the starch are granulated with water and dried. Magnesium stearate is added to the dried granules and the mixture is thoroughly blended. The blended mixture is compressed into a tablet.

### (C) Suppository

| Ingredients | Amount |
|---|---|
| Active compound | 150.0 mg |
| Theobromine sodium salicylate | 250.0 mg |
| Witepsol S55 | 1725.0 mg |

The inactive ingredients are mixed and melted. The active compound is then distributed in the molten mixture, poured into molds and allowed to cool.

### (D) Injection

| Ingredients | Amount |
|---|---|
| Active Compound | 20.0 mg |
| Buffering Agents | q.s. |
| Propylene glycol | 0.4 |
| Water for injection | 0.6 ml |

The active compound and buffering agents are dissolved in the propylene glycol at about 50^{o}C. The water for injection is then added with stirring and the resulting solution is filtered, filled into an ampule, sealed and sterilized by autoclaving.

### (E) Capsule

| Ingredients | Amount |
|---|---|
| Active Compound | 200.0 mg |
| Lactose | 450.0 mg |
| Magnesium stearate | 5.0 mg |

The finely ground active compound is mixed with the lactose and stearate and packed into a gelatin capsule.

## Claims

1. A compound of formula (I) 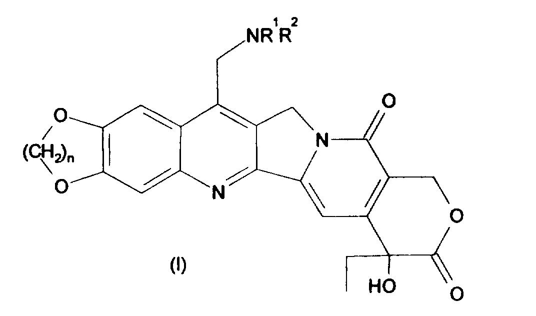 wherein:
n represents the integer 1 or 2; and
i) R¹ and R represent independently, hydrogen, (C₁₋₆)alkyl, (C₃₋₇)cycloalkyl, (C₃₋₇)cycloalkyl(C₁₋₆)alkyl, (C₃₋₆)alkenyl, hydroxy(C₁₋₆)alkyl, (C₁₋₆)alkoxy(C₁₋₆)alkyl;
ii) R¹ represents hydrogen, (C₁₋₆)alkyl, (C₃₋₇)cydoalkyl, (C₃₋₇)cycloalkyl(C₁₋₆)alkyl, (C₃₋₆)alkenyl, hydroxy(C₁₋₆)alkyl or (C₁₋₆)alkoxy(C₁₋₆)alkyl, and
R represents -COR³,
wherein:
R³ represents hydrogen, (C₁₋₆)alkyl, perhalo(C₁₋₆)alkyl, (C₃₋₇)cycloalkyl, (C₃₋₇)cycloalkyl(C₁₋₆)alkyl, (C₃₋₆)alkenyl, hydroxy(C₁₋₆)alkyl, (C₁₋₆)alkoxy, (C₁₋₆)alkoxy (C₁₋₆)alkyl; or
iii) R¹ and R taken together with the linking nitrogen form a saturated 3 to 7 atom heterocyclic group of formula (IA) 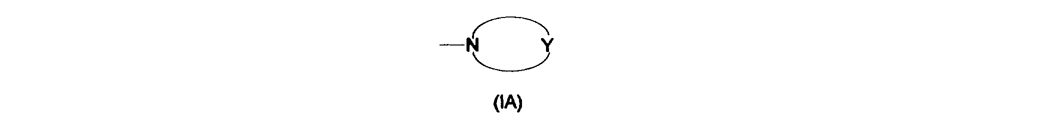 wherein:
Y represents O, S, SO, SO₂, CH₂ or NR⁴
wherein:
R⁴ represents hydrogen, (C₁₋₆)alkyl, perhalo(C₁₋₆)alkyl, aryl, aryl substituted with one or more
(C₁₋₆)alkyl, halogen, nitro, amino, (C₁₋₆)alkylamino, perhalo(C₁₋₆)alkyl, hydroxy(C₁₋₆)alkyl, (C₁₋₆)alkoxy, (C₁₋₆)alkoxy(C₁₋₆)alkyl groups; or
-COR⁵,
wherein:
R⁵ represents hydrogen, (C₁₋₆)alkyl, perhalo(C₁₋₆) alkyl, (C₁₋₆)alkoxy, aryl, aryl substituted with one or more
(C₁₋₆)alkyl, perhalo(C₁₋₆)alkyl, hydroxy(C₁₋₆)alkyl, (C₁₋₆)alkoxy(C₁₋₆)alkyl groups; and
the pharmaceutically acceptable salts and solvates thereof.

2. Compounds as claimed in Claim 1 wherein:
R¹ and R represent:
i) independently; hydrogen, (C₁₋₄)alkyl, (C₃₋₇)cycloalkyl, (C₃₋₇)cycloalkyl(C₁₋₄)alkyl, (C₃₋₄)alkenyl, hydroxy(C₁₋₄)alkyl, (C₁₋₄)alkoxy(C₁₋₄)alkyl, or
ii) taken together with the nitrogen form aziridine, azetidine, pyrrolidine, piperidine, hexamethylenimine, imidazolidine, pyrazolidine, isoxazolidine, piperazine, N-methylpiperazine, homopiperazine, N-methylhomopiperazine, thiazolidine, isothiazolidine, morpholine or thiomorpholine.

3. Compounds as claimed in Claim 1 or Claim 2 wherein:
R¹ and R are taken together to form a heterocyclic group selected from group consisting of aziridine, azetidine, pyrrolidine, piperidine, hexamethylenimine, imidazolidine, pyrazolidine, isoxazolidine, piperazine, N-methylpiperazine, homopiperazine, N-methylhomopiperazine, thiazolidine, isothiazolidine, morpholine or thiomorpholine.

4. Compounds as claimed in any of Claims 1 to 3 wherein:
n represents the integer 1 or 2; and
i) R¹ and R represent independently, hydrogen, (C₁₋₄)alkyl or hydroxy(C₁₋₄)alkyl;
ii) R¹ represents hydrogen and R represents -COR³, wherein R³ represents perhalo(C₁₋₄)alkyl; or
iii) R¹ and R taken together with the linking nitrogen form azetidine, pyrrolidine, piperidine, morpholine, thiomorpholine or piperazine optionally N-substituted with (C₁₋₄)alkyl; phenyl; phenyl substituted with one or more perhalo(C₁₋₄)alkyl or (C₁₋₄)alkoxy groups; or -COR⁵, wherein R⁵ represents (C₁₋₄)alkoxy.

5. A compound selected from:
7-Dimethylaminomethylene-10, 11-methylenedioxy-20(R,S)-camptothecin,
7-Dimethylaminomethylene-10, 11-methylenedioxy-20(S)-camptothecin,
7-Dimethylaminomethylene-10, 11-ethylenedioxy-20(R,S)-camptothecin,
7-Dimethylaminomethylene-10, 11-ethylenedioxy-20(S)-camptothecin,
7-Morpholinomethylene-10, 11-ethylenedioxy-20(R,S)-camptothecin,
7-Morpholinomethylene-10, 11-ethylenedioxy-20(S)-camptothecin,
7-Pyrrolidinomethylene-10, 11-ethylenedioxy-20(R,S)-camptothecin,
7-Piperidinomethylene-10, 11-methylenedioxy-20(R,S)-camptothecin,
7-Piperidinomethylene-10, 11-ethylenedioxy-20(R,S)-camptothecin,
7-(4-Methylpiperazinomethylene)-10, 11-ethylenedioxy-20(R,S)-camptothecin,
7-(4-Methylpiperazinomethylene)-10, 11-ethylenedioxy-20(S)-camptothecin,
7-Diethylaminomethylene-10, 11-methylenedioxy-20(S)-camptothecin,
7-Diethylaminomethylene-10, 11-ethylenedioxy-20(R,S)-camptothecin,
7-Diethylaminomethylene-10, 11-ethylenedioxy-20(S)-camptothecin,
7-N-Methylethanolaminomethylene-10, 11-methylenedioxy-20(R,S)-camptothecin,
7-N-Methylethanolaminomethylene-10, 11-ethylenedioxy-20(R,S)-camptotheci n,
7-Diethanolaminomethylene-10, 11-ethylenedfoxy-20(R,S)-camptothecin,
7-Diethanolaminomethylene-10, 11-ethylenedioxy-20(S)-camptothecin,
7-Azetidinomethylene-10, 11-methylenedioxy-20(R,S)-camptothecin
7-Azetidinomethylene-10, 11-methylenedioxy-20(S)-camptothecin
7-Thiomorpholinomethylene-10, 11-ethylenedioxy-20(S)-camptothecin,
7-Azetidinomethylene-10, 11-ethylenedioxy-20(S)-camptothecin,
7-(4-Methylpiperazinomethylene)-10, 11-methylenedioxy-20(S)-camptothecin,
7-Trifluoroacetamidomethylene-10, 11-ethylenedioxy-20(S)-camptothecin,
7-Trifluoroacetamidomethylene-10, 11-methylenedioxy-20(S)-camptothecin,
7-Aminomethylene-10, 11-ethylenedioxy-20(S)-camptothecin dihydrochloride,
7-Aminomethylene-10, 11-methylenedioxy-20(S)-camptothecin dihydrochloride,
7-tert-butyloxycarbonylene-piperazinomethylene-10,11-ethylenedioxy-20(S)-camptothecin,
7-Piperazinomethylene-10, 11-ethylenedioxy-(S)-camptothecin trifluoroacetic acid salt,
7-(α,α,α-trifluoro-m-tolyl)-piperazinomethylene-10,11-ethylenedioxy-20(S)-camptothecin,
7-(2-methoxyphenyl-piperazino)methylene-10, 11-ethylenedioxy-20(S)-camptothecin,
7-phenylpiperazinomethylene-10, 11-ethylenedioxy-20(S)-camptothecin, and pharmaceutically acceptable salts and solvates thereof.

6. 7-(4-Methylpiperazinomethylene)-10, 11-ethylenedioxy-20(S)camptothecin or a pharmaceutically acceptable salt or solvate thereof.

7. Compounds as claimed in any one of Claims 1 to 4 in the S configuration.

8. A compound of formula (I') 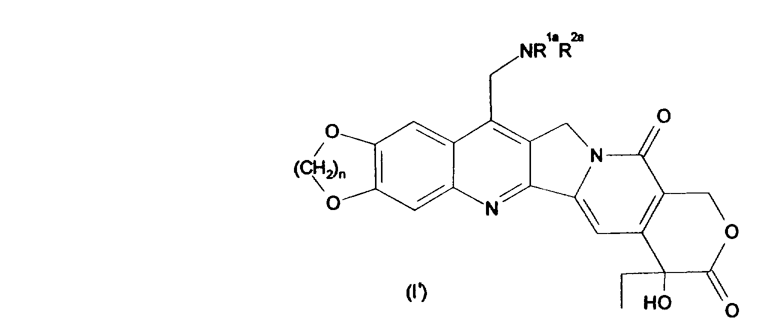 wherein:
n represents the integer 1 or 2; and
R^{1a} and R^{2a} represent:
i) independently, hydrogen, (C₁₋₆)alkyl, (C₃₋₇)cycloalkyl (C₃₋₇) cycloalkyl(C₁₋₆)alkyl, (C₃₋₆)alkenyl, hydroxy(C₁₋₆)alkyl, (C₁₋₆)alkoxy(C₁₋₆)alkyl, or
ii) taken together with the linking nitrogen form a 3 to 7 atom heterocyclic group of formula (IA') 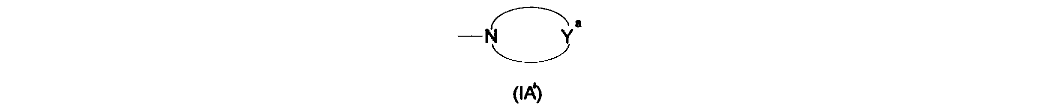 wherein;
Y^{a} represents O, S, CH₂, NH or N((C₁₋₆)alkyl), and the pharmaceutically acceptable salts thereof.

9. A compound as claimed in any one of claims 1 to 7 for use in therapy.

10. A pharmaceutical formulation comprising a compound as claimed in any one of claims 1 to 8 or a pharmaceutically acceptable salt or solvate thereof, together with a pharmaceutically acceptable carrier.

11. A pharmaceutical formulation comprising 7-(4-methylpiperazinomethylene)-10,11-ethylenedioxy-20(S)camptothecin or a pharmaceutically acceptable salt or solvate thereof, together with a pharmaceutically acceptable carrier.

12. The use of a compound as claimed in any one of Claims 1 to 8 in the preparation of a medicament for use in the treatment of tumours.

13. The use of 7-(4-methylpiperazinomethylene)-10,11-ethylenedioxy-20(S) camptothecin or a pharmaceutically acceptable salt or solvate thereof in the preparation of a medicament for use in the treatment of tumours.

14. A process for preparing a compound of formula (I) as defined in any one of claims 1 to 8 or a physiologically acceptable salt or solvate thereof which comprises
(a) reacting a compound of formula (IV) 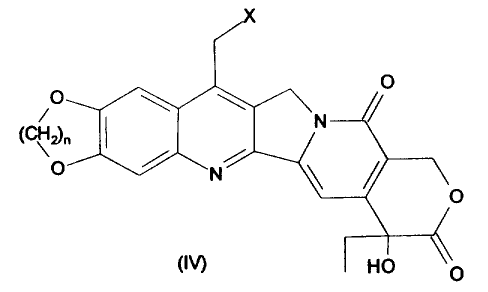 wherein X is halogen, with a compound of formula (V)
HNR¹R (V)
wherein R¹ and R are as defined for the compound of formula (I); or
(b) reacting a compound of formula (IIa) 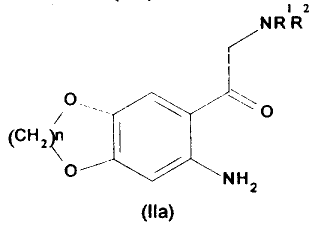 wherein R¹ and R are as defined for the compound of formula (I), with the compound of formula (III) 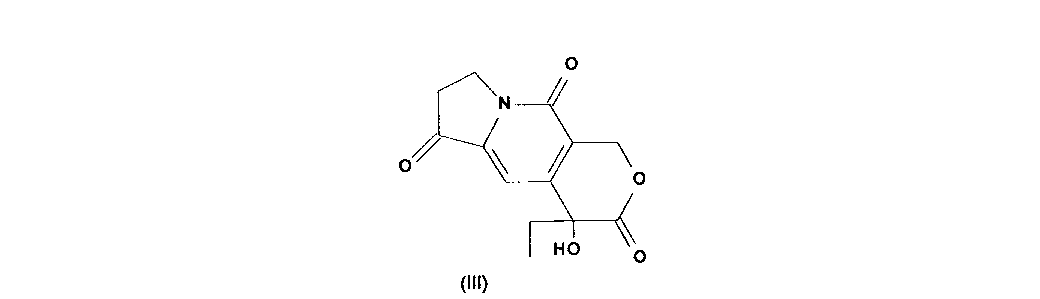 and if necessary and/or desired subjecting the compound thus obtained to one or more further reactions comprising:
(i) converting the resulting compound of formula (I) or a salt or protected derivative thereof into another compound of formula (I) and/or
(ii) removing any protecting group or groups and/or
(iii) converting a compound of formula (I) or a salt thereof into a physiologically acceptable salt or solvate thereof.

15. An alkali metal or alkaline-earth metal salt of the corresponding open E-ring form of a compound of formula (I) as defined in any one of Claims 1 to 8, obtainable by treatment of a compound of formula (I) as claimed in any one of claims 1 to 8 with an alkali metal or alkaline-earth metal base.

16. A compound of formula (IV) 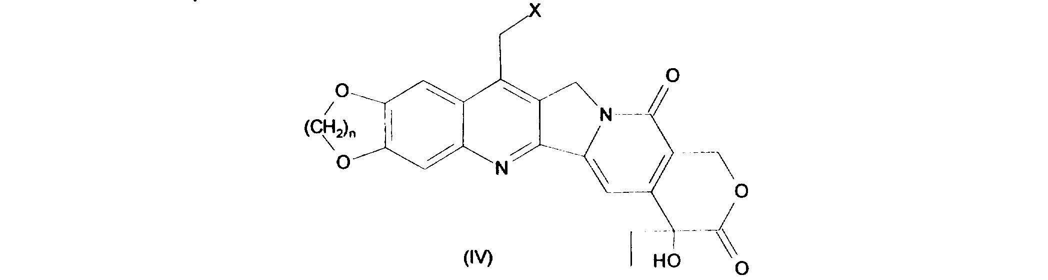 wherein n is 1 or 2 and X is halogen.

17. A compound selected from:
7-Chloromethyl-10,11-methylenedioxy-20(R,S)-camptothecin,
7-Chloromethyl-10,11-methylenedioxy-20(S)-camptothecin,
7-Chloromethyl-10,11-ethylenedioxy-20(R,S)-camptothecin and
7-Chloromethyl-10,11-ethylenedioxy-20(S)-camptothecin.

18. A compound of formula (IIA) 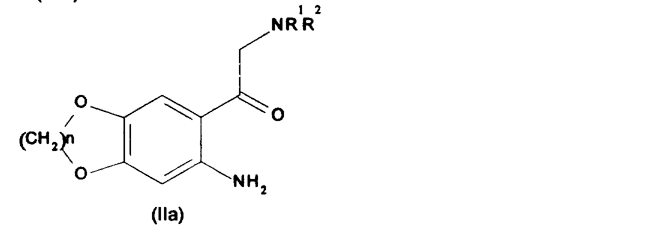 wherein:
n represents the integer 1 or 2; and
i) R¹ and R represent independently, hydrogen, (C₁₋₆)alkyl, (C₃₋₇)cycloalkyl, (C₃₋₇)cycloalkyl(C₁₋₆)alkyl, (C₃₋₆)alkenyl, hydroxy(C₁₋₆)alkyl, (C₁₋₆)alkoxy(C1-6)alkyl;
ii) R¹ represents hydrogen, (C₁₋₆)alkyl, (C₃₋₇)cycloalkyl, (C₃₋₇)cycloalkyl(C₁₋₆)alkyl, (C₃₋₆)alkenyl, hydroxy(C₁₋₆)alkyl or (C₁₋₆)alkoxy(C₁₋₆)alkyl, and
R represents -COR³,
wherein:
R³ represents hydrogen, (C₁₋₆)alkyl, perhalo(C₁₋₆)alkyl, (C₃₋₇)cycloalkyl, (C₃₋₇)cycloalkyl(C₁₋₆)alkyl, (C₃₋₆)alkenyl, hydroxy(C₁₋₆)alkyl, (C₁₋₆)alkoxy, (C₁₋₆)alkoxy(C₁₋₆)alkyl; or
iii) R¹ and R taken together with the linking nitrogen form a saturated 3 to 7 atom heterocyclic group of formula (IA) 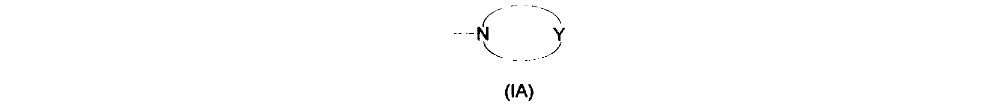 wherein:
Y represents O, S, SO, SO₂, CH₂ or NR⁴
wherein:
R⁴ represents hydrogen, (C₁₋₆)alkyl, perhalo(C₁₋₆)alkyl, aryl, aryl substituted with one or more
(C₁₋₆)alkyl, halogen, nitro, amino, (C₁₋₆)alkylamino, perhalo(C₁₋₆)alkyl, hydroxy(C₁₋₆)alkyl, (C₁₋₆)alkoxy, (C₁₋₆)alkoxy(C₁₋₆)alkyl groups; or
-COR⁵,
wherein:
R⁵ represents hydrogen, (C₁₋₆)alkyl, perhalo(C₁₋₆)alkyl, (C₁₋₆)alkoxy, aryl, aryl substituted with one or more (C₁₋₆)alkyl, perhalo(C₁₋₆)alkyl, hydroxy(C₁₋₆)alkyl or (C₁₋₆)alkoxy(C₁₋₆)alkyl groups.

19. The compound of Claim 18 wherein R¹ and R are taken together to form a heterocyclic group selected from group consisting of aziridine, azetidine, pyrrolidine, piperidine, hexamethylenimine, imidazolidine, pyrazolidine, isoxazolidine, piperazine, N-methylpiperazine, homopiperazine, N-methylhomopiperazine, thiazolidine, isothiazolidine, morpholine or thiomorpholine.

20. A compound of formula (II) 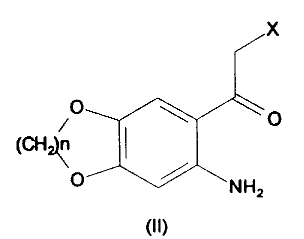 wherein n is the integer 1 or 2 and X is halogen.

## Patentansprüche

1. Verbindung der Formel (I): 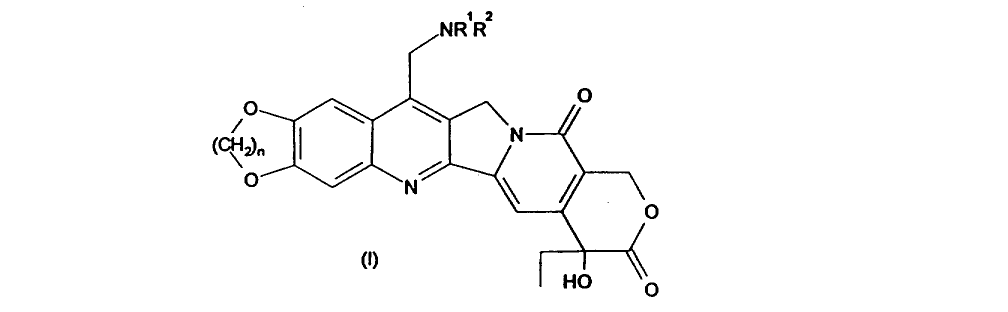 worin
n die ganze Zahl 1 oder 2 ist; und
i) R¹ und R unabhängig voneinander für Wasserstoff, (C₁₋₆)Alkyl, (C₃₋₇)Cycloalkyl, (C₃₋₇)Cycloalkyl(C₁₋₆)alkyl, (C₃₋₆)Alkenyl, Hydroxy(C₁₋₆)alkyl, (C₁₋₆)Alkoxy(C₁₋₆)alkyl stehen;
ii) R¹ für Wasserstoff, (C₁₋₆)Alkyl, (C₃₋₇)Cycloalkyl, (C₃₋₇)Cycloalkyl(C₁₋₆)alkyl, (C₃₋₆)Alkenyl, Hydroxy-(C₁₋₆)alkyl oder (C₁₋₆)Alkoxy(C₁₋₆)alkyl steht; und
R für -COR³ steht,
wobei R³ für Wasserstoff, (C₁₋₆)Alkyl, Perhalogen(C₁₋₆)alkyl, (C₃₋₇)Cycloalkyl, (C₃₋₇)Cycloalkyl(C₁₋₆)-alkyl, (C₃₋₆)Alkenyl, Hydroxy(C₁₋₆)alkyl, (C₁₋₆)Alkoxy, (C₁₋₆)Alkoxy(C₁₋₆)alkyl steht; oder
(iii) R¹ und R zusammen mit dem verbindenden Stickstoff eine gesättigte 3- bis 7atomige heterocyclische Gruppe der Formel (IA): 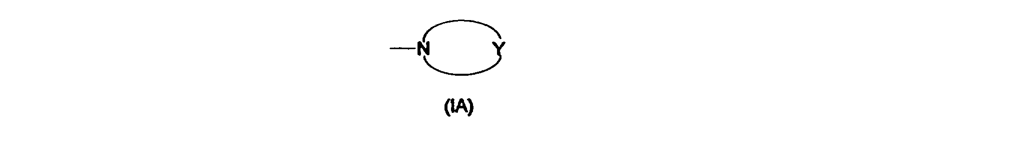 bilden, worin
Y für O, S, SO, SO₂, CH₂ oder NR⁴, wobei R⁴ für Wasserstoff, (C₁₋₆)Alkyl, Perhalogen(C₁₋₆)alkyl, Aryl, Aryl, das mit einer oder mehreren (C₁₋₆)Alkyl-, Halogen-, Nitro-, Amino-, (C₁₋₆)Alkylamino-, Perhalogen(C₁₋₆)alkyl-, Hydroxy-(C₁₋₆)alkyl-, (C₁₋₆)Alkoxy-, (C₁₋₆)Alkoxy(C₁₋₆)alkylgruppen substituiert ist, oder -COR⁵, wobei R⁵ für Wasserstoff, (C₁₋₆)Alkyl, Perhalogen(C₁₋₆)alkyl, (C₁₋₆)Alkoxy, Aryl, Aryl, das mit einer oder mehreren (C₁₋₆)Alkyl-, Perhalogen(C₁₋₆)-alkyl-, Hydroxy(C₁₋₆)alkyl-, (C₁₋₆)Alkoxy(C₁₋₆)alkylgruppen substituiert ist, steht;
und die pharmazeutisch annehmbaren Salze und Solvate davon.

2. Verbindungen nach Anspruch 1, dadurch **gekennzeichnet**, daß R¹ und R
i) unabhängig voneinander für Wasserstoff, (C₁₋₄)-Alkyl, (C₃₋₇)Cycloalkyl, (C₃₋₇)Cycloalkyl(C₁₋₄)alkyl, (C₃₋₄)Alkenyl, Hydroxy(C₁₋₄)alkyl, (C₁₋₄)Alkoxy(C₁₋₄)alkyl stehen; oder
ii) zusammen mit dem Stickstoff Aziridin, Azetidin, Pyrrolidin, Piperidin, Hexamethylenimin, Imidazolidin, Pyrazolidin, Isoxazolidin, Piperazin, N-Methylpiperazin, Homopiperazin, N-Methylhomopiperazin, Thiazolidin, Isothiazolidin, Morpholin oder Thiomorpholin bilden.

3. Verbindungen nach Anspruch 1 oder Anspruch 2, dadurch **gekennzeichnet**, daß R¹ und R zusammengenommen eine heterocyclische Gruppe, ausgewählt aus der Gruppe bestehend aus Aziridin, Azetidin, Pyrrolidin, Piperidin, Hexamethylenimin, Imidazolidin, Pyrazolidin, Isoxazolidin, Piperazin, N-Methylpiperazin, Homopiperazin, N-Methylhomopiperazin, Thiazolidin, Isothiazolidin, Morpholin oder Thiomorpholin, bilden.

4. Verbindungen nach einem der Ansprüche 1 bis 3, dadurch **gekennzeichnet**, daß n die ganze Zahl 1 oder 2 ist; und
i) R¹ und R unabhängig voneinander für Wasserstoff, (C₁₋₄)Alkyl oder Hydroxy(C₁₋₄)alkyl stehen;
ii) R¹ für Wasserstoff steht und R für -COR³ steht, wobei R³ für Perhalogen(C₁₋₄)alkyl steht; oder
iii) R¹ und R zusammen mit dem verbindenden Stickstoff Azetidin, Pyrrolidin, Piperidin, Morpholin, Thiomorpholin oder Piperazin, das gegebenenfalls mit (C₁₋₄)Alkyl, Phenyl, Phenyl, das mit einer oder mehreren Perhalogen-(C₁₋₄)alkyl- oder (C₁₋₄)Alkoxygruxyen substituiert ist, oder -COR⁵, wobei R⁵ für (C₁₋₄)Alkoxy steht, N-substituiert ist, bilden.

5. Verbindung, ausgewählt aus:
7-Dimethylaminomethylen-10,l1-methylendioxy-20(R,S)camptothecin,
7-Dimethylaminomethylen-10,11-methylendioxy-20(S)camptothecin,
7-Dimethylaminomethylen-10,11-ethylendioxy-20(R,S)camptothecin,
7-Dimethylaminomethylen-10,11-ethylendioxy-20(S)camptothecin,
7-Morpholinomethylen-10,11-ethylendioxy-20(R,S)camptothecin,
7-Morpholinomethylen-10,11-ethylendioxy-20(S)camptothecin,
7-Pyrrolidinomethylen-10,11-ethylendioxy-20(R,S)camptothecin,
7-Piperidinomethylen-10,11-methylendioxy-20(R,S)camptothecin,
7-Piperidinomethylen-10,11-ethylendioxy-20(R,S)camptothecin,
7-(4-Methylpiperazinomethylen)-10,11-ethylendioxy-20(R,S)-camptothecin,
7-(4-Methylpiperazinomethylen)-10,11-ethylendioxy-20(S)camptothecin,
7-Diethylaminomethylen-10,11-methylendioxy-20(S)camptothecin,
7-Diethylaminomethylen-10,11-ethylendioxy-20(R,S)camptothecin,
7-Diethylaminomethylen-10,11-ethylendioxy-20(S)camptothecin,
7-N-Methylethanolaminomethylen-10,11-methylendioxy-20(R,S)-camptothecin,
7-N-Methylethanolaminomethylen-10,11-ethylendioxy-20(R,S)-camptothecin,
7-Diethanolaminomethylen-10,11-ethylendioxy-20(R,S)camptothecin,
7-Diethanolaminomethylen-10,11-ethylendioxy-20(S)camptothecin,
7-Azetidinomethylen-10,11-methylendioxy-20(R,S)camptothecin,
7-Azetidinomethylen-10,11-methylendioxy-20(S)camptothecin,
7-Thiomorpholinomethylen-10,11-ethylendioxy-20(S)camptothecin,
7-Azetidinomethylen-10,11-ethylendioxy-20(S)camptothecin,
7-(4-Methylpiperazinomethylen)-10,11-methylendioxy-20(S)-camptothecin,
7-Trifluoracetamidomethylen-10,11-ethylendioxy-20(S)camptothecin,
7-Trifluoracetamidomethylen-10,11-methylendioxy-20(S)camptothecin,
7-Aminomethylen-10,11-ethylendioxy-20(S)camptothecin-dihydrochlorid,
7-Aminomethylen-10,11-methylendioxy-20(S)camptothecin-dihydrochlorid,
7-tert.-Butyloxycarbonylenpiperazinomethylen-10,11-ethylendioxy-20(S)camptothecin,
7-Piperazinomethylen-10,11-ethylendioxy-(S)camptothecin-trifluoressigsäuresalz,
7-(α,α,α-Trifluor-m-tolyl)piperazinomethylen-10,11-ethylendioxy-20(S)camptothecin,
7-(2-Methoxyphenylpiperazino)methylen-10,11-ethylendioxy-20(S)camptothecin,
7-Phenylpiperazinomethylen-10,11-ethylendioxy-20(S)camptothecin,
und die pharmazeutisch annehmbaren Salze und Solvate davon.

6. 7-(4-Methylpiperazinomethylen)-10,11-ethylendioxy-20(S)-camptothecin oder ein pharmazeutisch annehmbares Salz oder Solvat davon.

7. Verbindungen nach einem der Ansprüche 1 bis 4 in der S-Konfiguration.

8. Verbindung der Formel (I'): 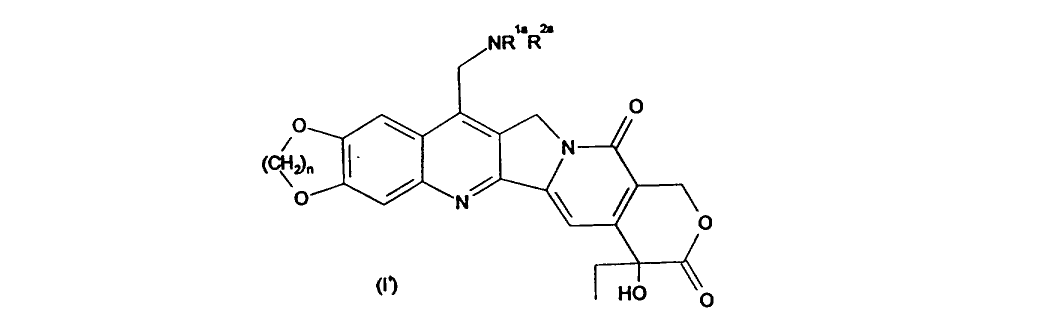 worin
n die ganze Zahl 1 oder 2 ist; und
R^{1a} und R^{2a}
i) unabhängig voneinander für Wasserstoff, (C₁₋₆)Alkyl, (C₃₋₇)Cycloalkyl, (C₃₋₇)Cycloalkyl(C₁₋₆)alkyl, (C₃₋₆)Alkenyl, Hydroxy(C₁₋₆)alkyl, (C₁₋₆)Alkoxy(C₁₋₆)alkyl stehen; oder
ii) zusammen mit dem verbindenden Stickstoff eine 3-bis 7atomige heterocyclische Gruppe der Formel (IA'): 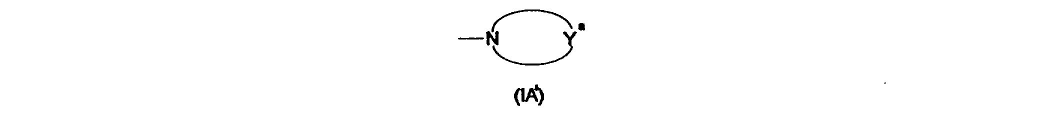 bilden, worin
Y^{a} für O, S, CH₂, NH oder N((C₁₋₆)alkyl) steht, und die pharmazeutisch annehmbaren Salze davon.

9. Verbindung nach einem der Ansprüche 1 bis 7 zur Verwendung in der Therapie.

10. Pharmazeutisches Präparat, umfassend eine Verbindung nach einem der Ansprüche 1 bis 8 oder ein pharmazeutisch annehmbares Salz oder Solvat davon zusammen mit einem pharmazeutisch annehmbaren Träger.

11. Pharmazeutisches Präparat, umfassend 7-(4-Methylpiperazinomethylen)-10,11-ethylendioxy-20(S)camptothecin oder ein pharmazeutisch annehmbares Salz oder Solvat davon zusammen mit einem pharmazeutisch annehmbaren Träger.

12. Verwendung einer Verbindung nach einem der Ansprüche 1 bis 8 zur Herstellung eines Arzneimittels zur Verwendung bei der Behandlung von Tumoren.

13. Verwendung von 7-(4-Methylpiperazinomethylen)-10,11-ethylendioxy-20(S)camptothecin oder eines pharmazeutisch annehmbaren Salzes oder Solvats davon zur Herstellung eines Arzneimittels zur Verwendung bei der Behandlung von Tumoren.

14. Verfahren zur Herstellung einer Verbindung der Formel (I) nach einem der Ansprüche 1 bis 8 oder eines physiologisch annehmbaren Salzes oder Solvats davon, umfassend
(a) die Umsetzung einer Verbindung der Formel (IV): 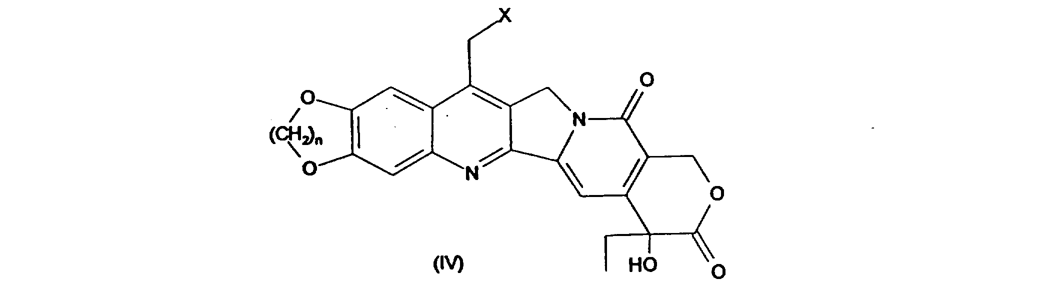
worin X für Halogen steht,
mit einer Verbindung der Formel (V):
HNR¹R (V)
worin R¹ und R wie im Zusammenhang mit der Verbindung der Formel (I) definiert sind; oder
b) die Umsetzung einer Verbindung der Formel (IIa): 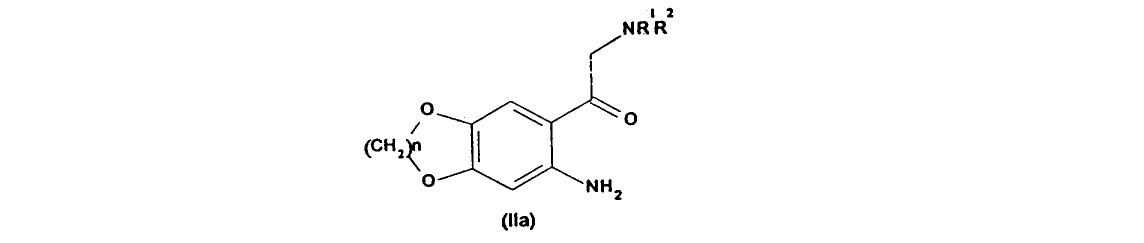
worin R¹ und R wie im Zusammenhang mit der Verbindung der Formel (I) definiert sind,
mit der Verbindung der Formel (III): 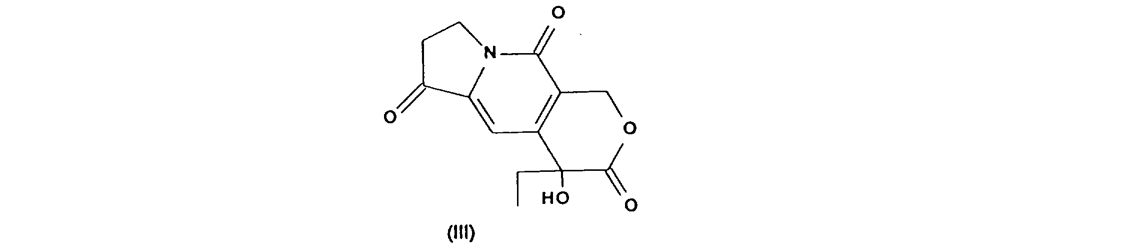 und erforderlichenfalls und/oder gewünschtenfalls die Unterwerfung der so erhaltenen Verbindung einer oder mehreren weiteren Reaktion(en), die folgendes umfaßt bzw. umfassen:
(i) Umwandlung der resultierenden Verbindung der Formel (I) oder eines Salzes oder geschützten Derivats davon in eine andere Verbindung der Formel (I) und/oder
(ii) Entfernung irgendeiner Schutzgruppe oder irgendwelcher Schutzgruppen und/oder
(iii) Umwandlung einer Verbindung der Formel (I) oder eines Salzes davon in ein physiologisch annehmbares Salz oder Solvat davon.

15. Alkalimetall- oder Erdalkalimetallsalz der entsprechenden offenen E-Ringform einer Verbindung der Formel (I) nach einem der Ansprüche 1 bis 8, erhältlich durch Behandlung einer Verbindung der Formel (I) nach einem der Ansprüche 1 bis 8 mit einer Alkalimetall- oder Erdalkalimetallbase.

16. Verbindung der Formel (IV): 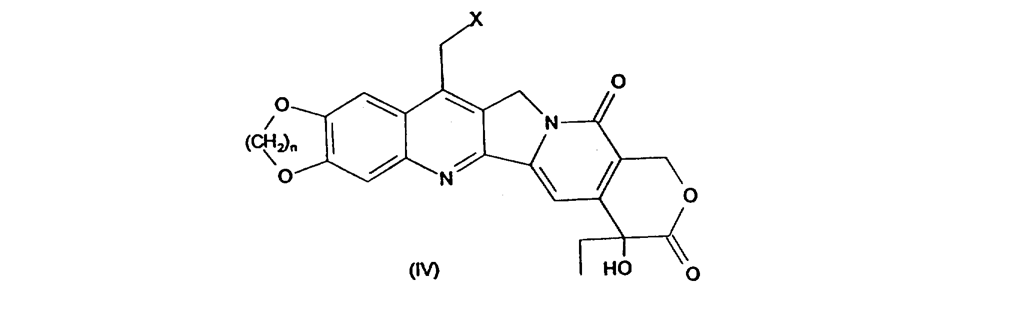 worin n den Wert 1 oder 2 hat und X für Halogen steht.

17. Verbindung, ausgewählt aus:
7-Chlormethyl-10,11-methylendioxy-20(R,S)camptothecin,
7-Chlormethyl-10,11-methylendioxy-20(S)camptothecin,
7-Chlormethyl-10,11-ethylendioxy-20(R,S)camptothecin und
7-Chlormethyl-10,11-ethylendioxy-20(S)camptothecin.

18. Verbindung der Formel (IIA): 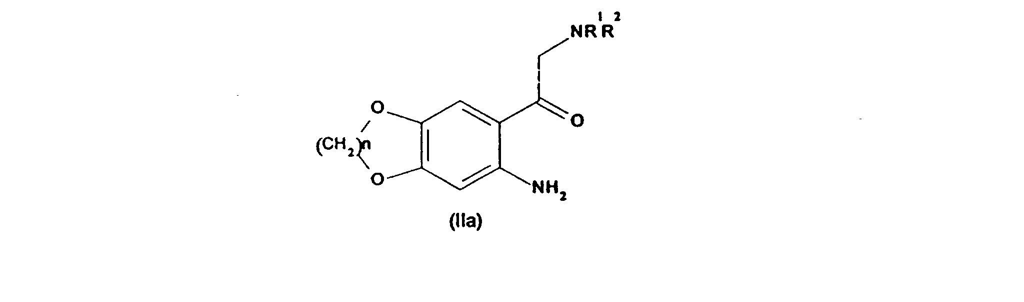 worin n die ganze Zahl 1 oder 2 ist; und
i) R¹ und R unabhängig voneinander für Wasserstoff, (C₁₋₆)Alkyl, (C₃₋₇)Cycloalkyl, (C₃₋₇)Cycloalkyl(C₁₋₆)alkyl, (C₃₋₆)Alkenyl, Hydroxy(C₁₋₆)alkyl, (C₁₋₆)Alkoxy(C₁₋₆)alkyl stehen;
ii) R¹ für Wasserstoff, (C₁₋₆)Alkyl, (C₃₋₇)Cycloalkyl, (C₃₋₇)Cycloalkyl(C₁₋₆)alkyl, (C₃₋₆)Alkenyl, Hydroxy(C₁₋₆)alkyl oder (C₁₋₆)Alkoxy(C₁₋₆)alkyl steht; und
R für -COR³ steht,
wobei R³ für Wasserstoff, (C₁₋₆)Alkyl, Perhalogen(C₁₋₆)alkyl, (C₃₋₇)Cycloalkyl, (C₃₋₇)Cycloalkyl(C₁₋₆)alkyl, (C₃₋₆)Alkenyl, Hydroxy(C₁₋₆)alkyl, (C₁₋₆)Alkoxy, (C₁₋₆)Alkoxy(C₁₋₆)alkyl steht; oder
(iii) R¹ und R zusammen mit dem verbindenden Stickstoff eine gesättigte 3- bis 7atomige heterocyclische Gruppe der Formel (IA): 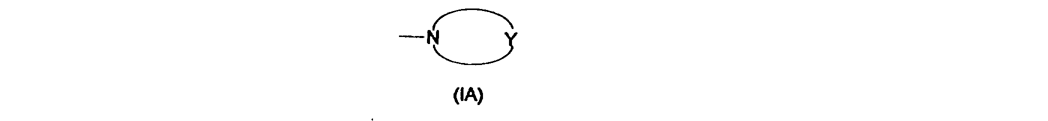 bilden, worin
Y für O, S, SO, SO₂, CH₂ oder NR⁴, wobei R⁴ für Wasserstoff, (C₁₋₆)Alkyl, Perhalogen(C₁₋₆)alkyl, Aryl, Aryl, das mit einer oder mehreren (C₁₋₆)Alkyl-, Halogen-, Nitro-, Amino-, (C₁₋₆)Alkylamino-, Perhalogen(C₁₋₆)alkyl-, Hydroxy(C₁₋₆)alkyl-, (C₁₋₆)Alkoxy-, (C₁₋₆)Alkoxy(C₁₋₆)alkylgruppen substituiert ist, oder -COR⁵, wobei R⁵ für Wasserstoff, (C₁₋₆)Alkyl, Perhalogen(C₁₋₆)alkyl, (C₁₋₆)Alkoxy, Aryl, Aryl, das mit einer oder mehreren (C₁₋₆)Alkyl-, Perhalogen(C₁₋₆)alkyl-, Hydroxy(C₁₋₆)alkyl-oder (C₁₋₆)Alkoxy(C₁₋₆)alkylgruppen substituiert ist, steht.

19. Verbindung nach Anspruch 18, dadurch **gekennzeichnet**, daß R¹ und R zusammengenommen eine heterocyclische Gruppe, ausgewählt aus der Gruppe bestehend aus Aziridin, Azetidin, Pyrrolidin, Piperidin, Hexamethylenimin, Imidazolidin, Pyrazolidin, Isoxazolidin, Piperazin, N-Methylpiperazin, Homopiperazin, N-Methylhomopiperazin, Thiazolidin, Isothiazolidin, Morpholin oder Thiomorpholin, bilden.

20. Verbindung der Formel (II): 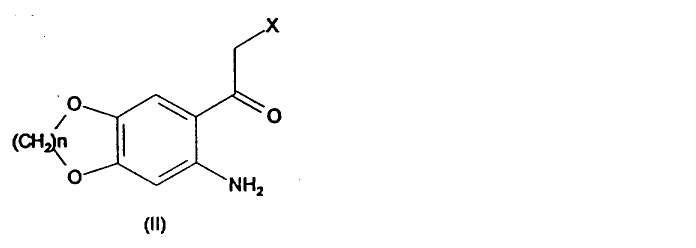 worin n die ganze Zahl 1 oder 2 ist und X für Halogen steht.

## Revendications

1. Composé de la formule (I) : 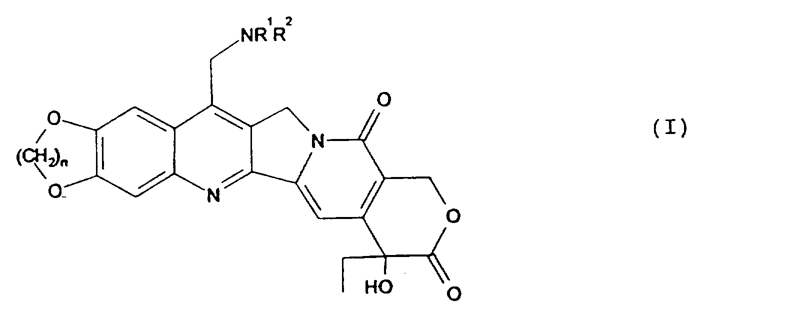 dans laquelle
n représente un nombre entier égal à 1 ou à 2, et
i) R¹ et R représentent, chacun indépendamment, un atome d'hydrogène, un radical alkyle(C₁-C₆), cycloalkyle(C₃-C₇) , cycloalkyle(C₃-C₇)alkyle(C₁-C₆), alcényle(C₃-C₆), hydroxyalkyle(C₁-C₆), alcoxy(C₁-C₆) alkyle(C₁-C₆) ;
ii) R¹ représente un atome d'hydrogène, un radical alkyle(C₁-C₆), cycloalkyle(C₃-C₇), cycloalkyle(C₃-C₇)alkyle(C₁-C₆), alcényle(C₃-C₆), hydroxyalkyle(C₁-C₆), ou alcoxy(C₁-C₆)alkyle(C₁-C₆), et
R représente -COR³,
où
R³ représente un atome d'hydrogène, un radical alkyle(C₁-C₆), perhalo-alkyle(C₁-C₆), cycloalkyle(C₃-C₇) , cycloalkyle(C₃-C₇)alkyle(C₁-C₆), alcényle(C₃-C₆), hydroxyalkyle(C₁-C₆), alcoxy(C₁-C₆) , alcoxy(C₁-C₆)alkyle(C₁-C₆); ou bien
iii) R¹ et R, considérés ensemble avec l'atome d'azote qui les lie, forment un groupe hétérocyclique saturé comportant de 3 à 7 atomes et répondant à la formule (IA) : 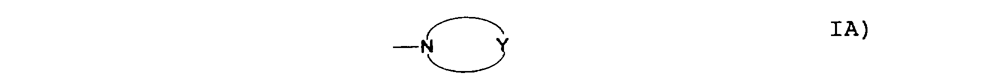 dans laquelle
Y représente O, S, SO, SO₂, CH₂ ou NR⁴, où
R⁴ représente un atome d'hydrogène, un radical alkyle(C₁-C₆), perhalo-alkyle(C₁-C₆), aryle, aryle substitué par un ou plusieurs
radicaux alkyle(C₁-C₆), atomes d'halogène, radicaux nitro, amino, alkyle(C₁-C₆)amino, perhalo-alkyle(C₁-C₆), hydroxyalkyle(C₁-C₆), alcoxy(C₁-C₆), alcoxy(C₁-C₆)alkyle(C₁-C₆) ; ou
-COR⁵
où
R⁵ représente un atome d'hydrogène, un radical alkyle(C₁-C₆), perhalo-alkyle(C₁-C₆), alcoxy(C₁-C₆), aryle, aryle substitué par un ou plusieurs
radicaux alkyle(C₁-C₆), perhalo-alkyle(C₁-C₆), hydroxyalkyle(C₁-C₆), alcoxy(C₁-C₆)alkyle(C₁-C₆), et
les solvates et sels pharmaceutiquement acceptables du composé en question.

2. Composés suivant la revendication 1, dans lesquels :
R¹ et R représentent :
i) chacun indépendamment, un atome d'hydrogène, un radical alkyle(C₁-C₄) , cycloalkyle(C₃-C₇) , cycloalkyle(C₃-C₇) alkyle(C₁-C₄), alcényle(C₃-C₄), hydroxyalkyle(C₁-C₄), alcoxy(C₁-C₄)alkyle(C₁-C₄), ou bien
ii) considérés ensemble avec l'atome d'azote, ils forment l'une des substances qui suivent : l'aziridine, l'azétidine, la pyrrolidine, la pipéridine, l'hexaméthylèneimine, l'imidazolidine, la pyrazolidine, l'isoxazolidine, la pipérazine, la N-méthylpipérazine, l'homopipérazine, la N-méthylhomopipérazine, la thiazolidine, l'isothiazolidine, la morpholine et la thiomorpholine.

3. Composés suivant la revendication 1 ou la revendication 2, dans lesquels :
R¹ et R considérés ensemble, forment un radical hétérocyclique choisi dans le groupe constitué des systèmes hétérocycliques suivants : l'aziridine, l'azétidine, la pyrrolidine, la pipéridine, l'hexaméthylèneimine, l'imidazolidine, la pyrazolidine, l'isoxazolidine, la pipérazine, la N-méthylpipérazine, l'homopipérazine, la N-méthylhomopipérazine, la thiazolidine, l'isothiazolidine, la morpholine et la thiomorpholine.

4. Composés suivant l'une quelconque des revendications 1 à 3, dans lesquels :
n représente un nombre entier dont la valeur est égale à 1 ou à 2, et
i) R¹ et R représentent, chacun indépendamment, un atome d'hydrogène, un radical alkyle(C₁-C₄) ou hydroxyalkyle(C₁-C₄) ;
ii) R¹ représente un atome d'hydrogène et R représente le radical -COR³, où R³ représente un groupe perhalo-alkyle(C₁-C₄), ou bien
iii) R¹ et R, considérés ensemble avec l'atome d'azote qui les lie, forment l'une des substances suivantes : azétidine, pyrrolidine, pipéridine, morpholine, thiomorpholine et pipérazine, lesquelles substances sont éventuellement N-substituées par des radicaux alkyle(C₁-C₄), phényle, phényle substitué par un ou plusieurs groupes perhalo-alkyle(C₁-C₄) ou alcoxy(C₁-C₄), ou -COR⁵, où R⁵ représente un groupe alcoxy(C₁-C₄).

5. Composé choisi parmi les suivants :
7-Diméthylaminométhylène-10,11-méthylènedioxy-20(R,S)-camptothécine,
7-Diméthylaminométhylène-10,11-méthylènedioxy-20(S)-camptothécine,
7-Diméthylaminométhylène-10,11-éthylènedioxy-20(R,S)-camptothécine,
7-Diméthylaminométhylène-10,11-éthylènedioxy-20(S)-camptothécine,
7-Morpholinométhylène-10,11-éthylènedioxy-20(R,S)-camptothécine,
7-Morpholinométhylène-10,11-éthylènedioxy-20(S)-camptothécine,
7-Pyrrolidinométhylène-10,11-éthylènedioxy-20(R,S)camptothécine,
7-Pipéridinométhylène-10,11-méthylènedioxy-20(R,S)camptothécine,
7-Pipéridinométhylène-10,11-éthylènedioxy-20(R,S)-camptothécine,
7-(4-Méthylpipérazinométhylène)-10,11-éthylènedioxy-20(R,S)-camptothécine,
7-(4-Méthylpipérazinométhylène)-10,11-éthylènedioxy-20(S)-camptothécine,
7-Diéthylaminométhylène-10,11-méthylènedioxy-20(S)-camptothécine,
7-Diéthylaminométhylène-10,11-éthylènedioxy-20(R,S)-camptothécine,
7-Diéthylaminométhylène-10,11-éthylènedioxy-20(S)-camptothécine,
7-N-Méthyléthanolaminométhylène-10,11-méthylènedioxy-20(R,S)-camptothécine,
7-N-Méthyléthanolaminométhylène-10,11-éthylènedioxy-20(R,S)-camptothécine,
7-Diéthanolaminométhylène-10,11-éthylènedioxy-20(R,S)-camptothécine,
7-Diéthanolaminométhylène-10,11-éthylènedioxy-20(S)-camptothécine,
7-Azétidinométhylène-10,11-méthylènedioxy-20(R,S)-camptothécine,
7-Azétidinométhylène-10,11-méthylènedioxy-20(S)-camptothécine,
7-Thiomorpholinométhylène-10,11-éthylènedioxy-20(S)-camptothécine,
7-Azétidinométhylène-10,11-éthylènedioxy-20(S)-camptothécine,
7-(4-Méthylpipérazinométhylène)-10,11-méthylènedioxy-20(S)-camptothécine,
7-Trifluoracétamidométhylène-10,11-éthylènedioxy-20(S)-camptothécine,
7-Trifluoracétamidométhylène-10,11-méthylènedioxy-20(S)camptothécine,
7-Aminométhylène-10,11-éthylènedioxy-20(S)-camptothécine dichlorhydrate,
7-Aminométhylène-10,11-méthylènedioxy-20(S)-camptothécine dichlorhydrate,
7-t-Butyloxycarbonylpipérazinométhylène-10,11-éthylènedioxy-20(S)-camptothécine,
7-Pipérazinométhylène-10,11-éthylènedioxy-(S)-camptothécine sel avec l'acide trifluoracétique,
7-(α,α,α-Trifluoro-m-tolyl)-pipérazinométhylène-10,11-éthylènedioxy-20(S)-camptothécine,
7-(2-Méthoxyphénylpipérazino)méthylène-10,11-éthylènedioxy-20(S)-camptothécine, et
7-Phénylpipérazinométhylène-10,11-éthylènedioxy-20(S)-camptothécine,
et les solvates et sels pharmaceutiquement acceptables de ces composés.

6. 7-(4-Méthylpipérazinométhylène)-10,11-éthylènedioxy-20(S)camptothécine ou un solvate ou sel pharmaceutiquement acceptable de celle-ci.

7. Composés suivant l'une quelconque des revendications 1 à 4 en configuration S.

8. Composé de la formule (I') : 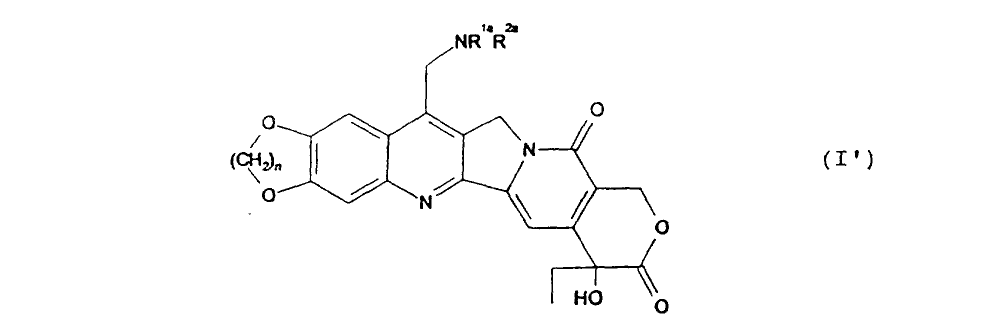 dans laquelle
n est un nombre entier égal à 1 ou à 2, et
R^{1a} et R^{2a} représentent :
i) chacun indépendamment, un atome d'hydrogène, un radical alkyle(C₁-C₆), cycloalkyle(C₃-C₇), cycloalkyle(C₃-C₇) alkyle(C₁-C₆), alcényle(C₃-C₆), hydroxyalkyle(C₁-C₆), alcoxy(C₁-C₆)alkyle(C₁-C₆), ou bien
ii) considérés ensemble avec l'atome d'azote qui les lie, ils forment un radical hétérocyclique comportant 3 à 7 atomes, de la formule (IA') : 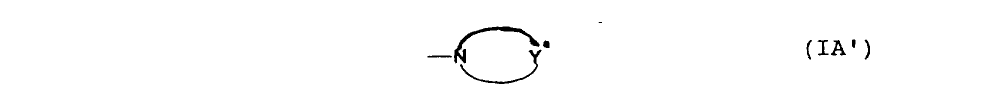 dans laquelle
Y^{a} représente O, S, CH₂, NH ou N[alkyle(C₁-C₆)], et
les sels pharmaceutiquement acceptables de ce composé.

9. Composé suivant l'une quelconque des revendications 1 à 7, à utiliser dans le domaine thérapeutique.

10. Composition pharmaceutique comprenant un composé suivant l'une quelconque des revendications 1 à 8, ou un solvate ou un sel pharmaceutiquement acceptable d'un tel composé, en même temps qu'un véhicule ou excipient pharmaceutiquement acceptable.

11. Composition pharmaceutique comprenant de la 7-(4-méthylpipérazinométhylène)-10,11-éthylènedioxy-20(S)camptothécine ou un solvate ou un sel pharmaceutiquement acceptable de celle-ci, en même temps qu'un véhicule ou excipient pharmaceutiquement acceptable.

12. Utilisation d'un composé suivant l'une que quelconque des revendications 1 à 8, pour la préparation d'un médicament destiné au traitement de tumeurs.

13. Utilisation de la 7-(4-méthylpipérazinométhylène)-10,11-éthylènedioxy-20(S)-camptothécine, ou d'un solvate ou d'un sel pharmaceutiquement acceptable de celle-ci en vue de la préparation d'un médicament destiné au traitement de tumeurs.

14. Procédé de préparation d'un composé de la formule (I) tel que défini dans l'une quelconque des revendications 1 à 8, ou d'un solvate ou d'un sel physiologiquement acceptable d'un tel composé, caractérisé en ce que :
(a) on fait réagir un composé de la formule (IV) : 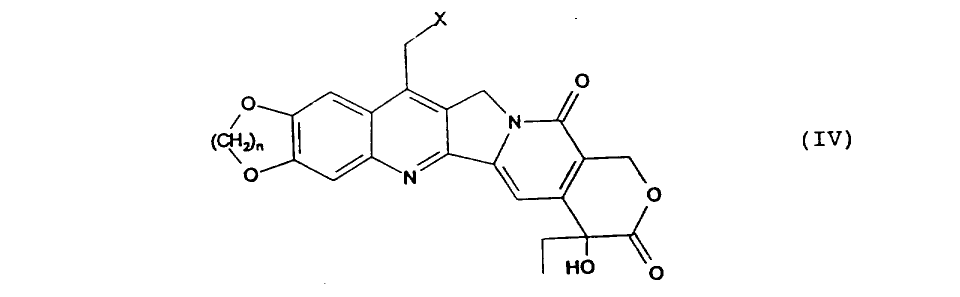 dans laquelle X représente un atome d'halogène, avec un composé de la formule (V) :
HNR¹R (V)
dans laquelle R¹ et R possèdent les significations qui leur ont été attribuées à propos de la définition du composé de la formule (I); ou
(b) on fait réagir un composé de la formule (IIa) : 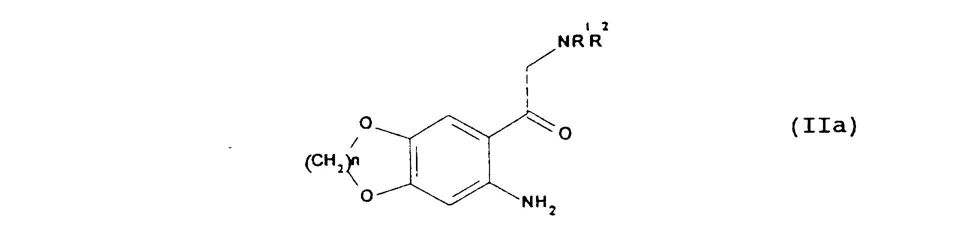 dans laquelle R¹ et R possèdent les significations qui leur ont été attribuées à propos de la définition de la formule (I), avec le composé de la formule (III) : 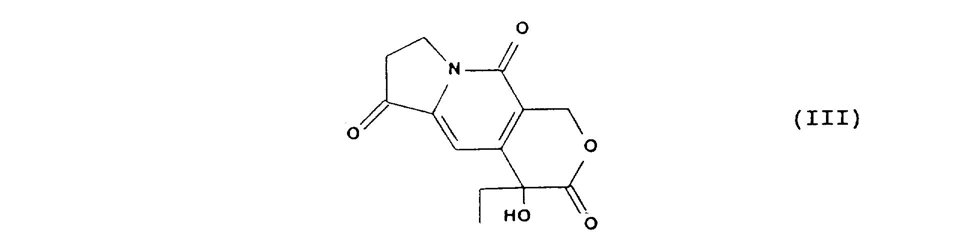 et, si cela se révèle nécessaire et/ou souhaitable, on soumet le composé ainsi obtenu à une ou plusieurs réactions supplémentaires qui comprennent :
(i) la conversion du composé de la formule (I) ainsi obtenu ou d'un sel ou d'un dérivé protégé de ce composé en un autre composé de la formule (I), et/ou
(ii) l'élimination de n'importe quel groupe ou de n'importe quels groupes protecteurs, et/ou
(iii) la conversion d'un composé de la formule (I) ou d'un sel de celui-ci en un solvate ou un sel physiologiquement acceptable de ce composé.

15. Sel de métal alcalin ou de métal alcalino-terreux de la forme à noyau E ouvert correspondante d'un composé de la formule (I) tel que défini dans l'une quelconque des revendications 1 à 8, que l'on peut obtenir par le traitement d'un composé de la formule (I) suivant l'une quelconque des revendications 1 à 8, par une base à métal alcalin ou à métal alcalino-terreux.

16. Composé de la formule (IV) : 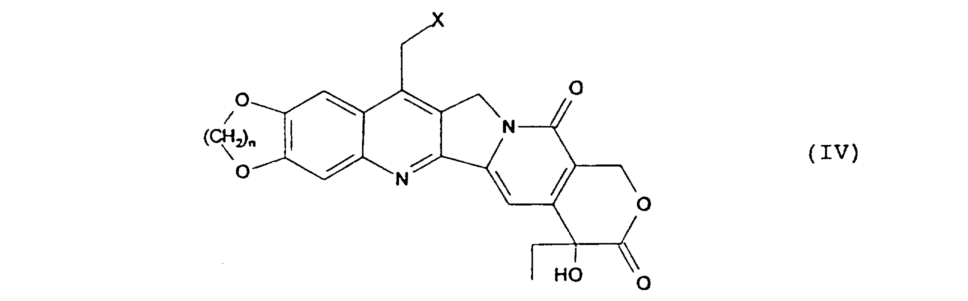 dans laquelle n est égal à 1 ou à 2 et X représente un atome d'halogène.

17. Composé choisi parmi les substances suivantes :
7-chlorométhyl-10,11-méthylènedioxy-20(R,S)-camptothécine,
7-chlorométhyl-10,11-méthylènedioxy-20(S)-camptothécine,
7-chlorométhyl-10,11-éthylènedioxy-20(R,S)-camptothécine, et
7-chlorométhyl-10,11-éthylènedioxy-20(S)-camptothécine.

18. Composé de la formule (IIA) : 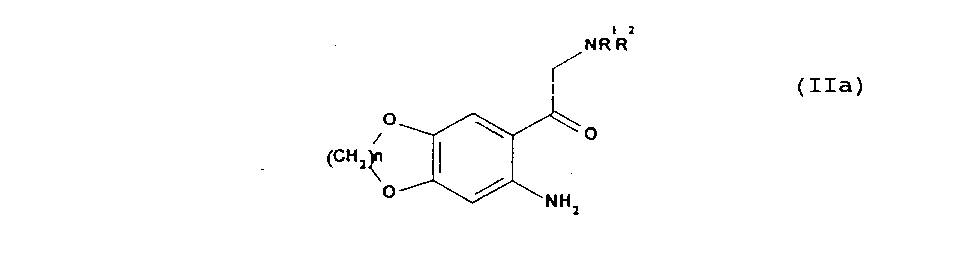 dans laquelle
n représente un nombre entier égal à 1 ou à 2, et
i) R¹ et R représentent, chacun indépendamment, un atome d'hydrogène, un radical alkyle(C₁-C₆), cycloalkyle(C₃-C₇), cycloalkyle(C₃-C₇) alkyle(C₁-C₆), alcényle(C₃-C₆), hydroxyalkyle(C₁-C₆), alcoxy(C₁-C₆)alkyle(C₁-C₆);
ii) R¹ représente un atome d'hydrogène, un radical alkyle(C₁-C₆), cycloalkyle(C₃-C₇), cycloalkyle(C₃-C₇)alkyle(C₁-C₆), alcényle(C₃-C₆), hydroxyalkyle(C₁-C₆), ou alcoxy(C₁-C₆)alkyle(C₁-C₆), et
R représente -COR³,
où
R³ représente un atome d'hydrogène, un radical alkyle(C₁-C₆), perhalo-alkyle(C₁-C₆), cycloalkyle(C₃-C₇), cycloalkyle(C₃-C₇)alkyle(C₁-C₆), alcényle(C₃-C₆), hydroxyalkyle(C₁-C₆), alcoxy(C₁-C₆), alcoxy(C₁-C₆) alkyle(C₁-C₆) ;
ou bien
iii) R¹ et R, considérés ensemble avec l'atome d'azote qui les lie, forment un groupe hétérocyclique saturé comportant de 3 à 7 atomes et répondant à la formule (IA) : 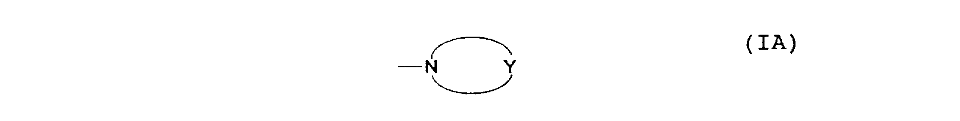 dans laquelle
Y représente O, S, SO, SO₂, CH₂ ou NR⁴, où
R⁴ représente un atome d'hydrogène, un radical alkyle(C₁-C₆), perhalo-alkyle(C₁-C₆), aryle, aryle substitué par un ou plusieurs
radicaux alkyle(C₁-C₆), atomes d'halogène, radicaux nitro, amino, alkyle(C₁-C₆)amino, perhalo-alkyle(C₁-C₆), hydroxyalkyle(C₁-C₆), alcoxy(C₁-C₆), alcoxy(C₁-C₆) alkyle(C₁-C₆); ou
-COR⁵
où
R⁵ représente un atome d'hydrogène, un radical alkyle(C₁-C₆), perhalo-alkyle(C₁-C₆), alcoxy(C₁-C₆), aryle, aryle substitué par un ou plusieurs
radicaux alkyle(C₁-C₆), perhalo-alkyle(C₁-C₆), hydroxyalkyle(C₁-C₆), alcoxy(C₁-C₆)alkyle(C₁-C₆).

19. Composé suivant la revendication 18, dans lequel R¹ et R considérés ensemble forment un radical hétérocyclique choisi dans le groupe formé par les substances suivantes : l'aziridine, l'azétidine, la pyrrolidine, la pipéridine, l'hexaméthylèneimine, l'imidazolidine, la pyrazolidine, l'isoxazolidine, lapipérazine, la N-méthylpipérazine, l'homopipérazine, la N-méthylhomopipérazine, la thiazolidine, l'isothiazolidine, la morpholine et la thiomorpholine.

20. Composé de la formule (II) : 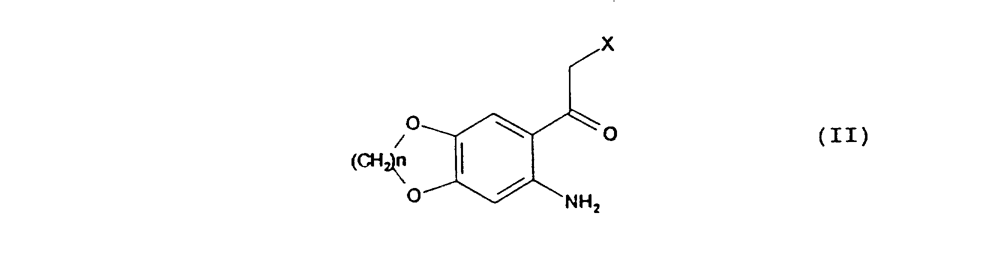 dans laquelle n est égal à 1 ou à 2 et X représente un atome d'halogène.
